(19) Europäisches Patentamt / European Patent Office / Office européen des brevets

(11) EP 1 753 747 B1

(12) # FASCICULE DE BREVET EUROPEEN

(45) Date de publication et mention de la délivrance du brevet: **13.01.2010 Bulletin 2010/02**

(21) Numéro de dépôt: **05769495.2**

(22) Date de dépôt: **04.05.2005**

(51) Int Cl.: ***C07D 403/10*** (2006.01) ***A61K 31/53*** (2006.01)

(86) Numéro de dépôt international: **PCT/FR2005/001132**

(87) Numéro de publication internationale: **WO 2005/121128 (22.12.2005 Gazette 2005/51)**

(54) **DERIVES DIMERIQUES DE 5,6-DIPHENYL-1,2,4-TRIAZINE ET LEURS UTILISATIONS EN TANT QU'AGENTS PROTECTEURS DU SOLEIL**

DIMERE 5,6-DIPHENYL-1,2,4-TRIAZIN-DERIVATE UND DEREN VERWENDUNG IN FORM VON SONNENSCHUTZMITTELN

5,6-DIPHENYL-1,2,4-TRIAZINIC DIMERIC DERIVATIVES AND THE USE THEREOF IN THE FORM OF SUN-PROTECTIVE AGENTS

(84) Etats contractants désignés:
**AT BE BG CH CY CZ DE DK EE ES FI FR GB GR HU IE IS IT LI LT LU MC NL PL PT RO SE SI SK TR**

(30) Priorité: **05.05.2004 FR 0404811**

(43) Date de publication de la demande: **21.02.2007 Bulletin 2007/08**

(73) Titulaires:
- **Pierre Fabre Dermo-Cosmétique 92100 Boulogne-Billancourt (FR)**
- **CENTRE NATIONAL DE LA RECHERCHE SCIENTIFIQUE (CNRS) 75016 Paris (FR)**
- **UNIVERSITE CLAUDE BERNARD - LYON 1 69100 Villeurbanne (FR)**

(72) Inventeurs:
- **PICOUL, Willy F-69004 Lyon (FR)**
- **CIUFOLINI, Marco Vancouver, BC V6K 1V5 (CA)**
- **BORDAT, Pascal F-31320 Mervilla (FR)**
- **TARROUX, Roger F-31300 Toulouse (FR)**

(74) Mandataire: **Ahner, Francis et al Cabinet Régimbeau 20, rue de Chazelles 75847 Paris cedex 17 (FR)**

(56) Documents cités:
**EP-A- 0 818 450** **DD-A- 247 899**
**US-A- 3 211 729**

- **CASE, F.H.: "The Preparation of 2,4- and 2,6-Bis-Triazinyl and Triazolinyl Derivatives of Pyridine" J. HETEROCYCL. CHEM., vol. 8, 1971, pages 1043-1046, XP002308683**
- **WAHL, B.; WÖHRLE, D.: "Über Poly(1,3,5-triazin)e und Poly(1,2,4-triazin)e" DIE MAKROMOLEKULARE CHEMIE, vol. 176, 1975, pages 849-858, XP002308684**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention se rapporte à des dérivés de 5,6-diphényl-1,2,4-triazine, et en particulier à leur utilisation comme filtres solaires sur la peau humaine et les cheveux ou comme agents protecteurs de la lumière dans l'industrie de matériaux synthétiques tels que les plastiques, les verres, les textiles. La présente invention a également pour objet les compositions cosmétiques contenant lesdits dérivés.

On rappellera brièvement que l'action du rayonnement solaire sur la peau dépend essentiellement de l'énergie des radiations qui atteignent les différentes couches cutanées. De façon générale, les radiations les plus énergétiques, i.e. possédant la longueur d'onde la plus faible ($E=hc/\lambda$), provoquent des érythèmes ou « coup de soleil », alors que les radiations moins énergétiques n'engendrent qu'un simple brunissage de la peau. On considère donc qu'un filtre solaire, destiné à entrer dans la composition des préparations cosmétologiques dites « anti-solaires » doit absorber au maximum les radiations de faible longueur d'onde, tout en restant transparent aux radiations de plus grande longueur d'onde.

Les photobiologistes ont pour habitude de diviser le spectre ultra-violet en trois portions appelées UV-A, UV-B et UV-C correspondant aux gammes de longueurs d'ondes décroissantes respectivement comprises entre 400 nm et 320 nm, enture 320 nm et 280 nm et entre 280 nm et 200 nm.

Les UV-B et UV-A permettent le brunissement de l'épiderme humain. Les UV-B provoquent des érythèmes et des brûlures cutanées qui peuvent nuire au développement du bronzage naturel. Pour ces raisons, ainsi que pour des raisons esthétiques, il existe une demande constante de moyens de contrôle de ce bronzage naturel en vue de contrôler la couleur de la peau. Il convient donc de filtrer ce rayonnement UV-B.

On sait également que les rayons UV-A, sont susceptibles d'induire une altération de la peau, notamment dans le cas des peaux sensibles ou des peaux continuellement exposées au rayonnement solaire. Les rayons UV-A provoquent en particulier une perte d'élasticité de la peau et l'apparition de rides conduisant à un vieillissement prématuré. Ils provoquent le déclenchement de réaction érythémateuse
ou amplifient cette réaction chez certains sujets et peuvent même être à l'origine de réactions phototoxiques ou photoallergiques. Il est donc souhaitable de filtrer aussi le rayonnement UV-A.

Les UV-C, les plus fortement énergétiques créent des photokératites. L'ozone formé dans la stratosphère absorbe généralement une grande partie de ce rayonnement UV-C que l'on retrouve en revanche en quantité importante dans les rayonnements émis par les lampes artificielles, souvent responsables de graves accidents cutanés. Les UV-B qui pénètrent dans la couche de la peau et en particulier dans le corps muqueux de l'épiderme, provoquent des érythèmes solaires. Par suite, l'ensemble des radiations (UV-B + UV-C) constitue ce que l'on appelle la bande érythémale à l'égard de laquelle les filtres solaires doivent jouer le rôle d'écran. Les UV-A produisent la pigmentation directe de la peau (mélanogénèse), i.e. le brunissement de la peau.

Des composés dérivés de benzotriazoles et/ou benzothiazoles sont connus en tant que filtres UV, notamment dans le domaine cosmétique. La demande de brevet FR 2 803 194 décrit ainsi des dérivés de S-triazines portant des groupements phénylbenzothiazoles ou benzothiazoles utiles en tant que filtres UV sous forme particulaire. Ces composés couvrent le domaine de l'UV-A et de l'UV-B mais ils présentent l'inconvénient majeur d'absorber dans le visible (longueurs d'onde supérieure à 400 nm). Ces produits sont donc fortement colorés, ce qui limite leur utilisation dans les produits cosmétiques.

La présente invention propose de nouveaux dérivés de 5,6-diphényl-1,2,4-triazine capables d'absorber dans le domaine des UV-A et/ou des UV-B et/ou des UV-C, sans absorber dans le domaine du visible. Ces composés présentent donc l'avantage d'être peu colorés.

Ils présentent aussi l'avantage de pouvoir être spécifiques de l'un de ces domaines spectraux. Ceci est intéressant lorsque l'on souhaite filtrer un domaine UV précis (UV-A, UV-B ou UV-C), pour compléter par exemple l'efficacité spectrale d'un filtre UV qui présente une lacune sur ce domaine particulier.

Ces nouveaux dérivés offrent ainsi une gammé variée de filtres UV spécifiques et qui peuvent aussi présenter différents degrés d'absorbance. La combinaison de plusieurs de ces filtres sélectionnés en fonction de leur spécificité et leur degré d'absorbance, permet donc de préparer toute sorte de filtres UV agissant dans le spectre et avec l'absorbance désirés.

Ces nouveaux dérivés présentent aussi l'avantage d'être solubles dans divers excipients pharmaceutiquement acceptables, et de présenter une photostabilité meilleure que certains filtres commerciaux, ce qui les rend particulièrement utiles dans les produits cosmétiques, notamment les protections solaires.

La présente invention a pour objet les composés 5,6-diphényl-1,2,4-triaziniques de formule générale (I) :

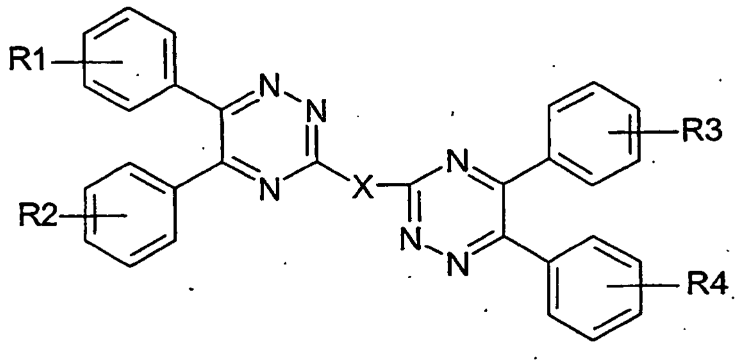

(I)

dans laquelle :

- $R_1$, $R_2$, $R_3$ et $R_4$, identiques ou différents, représentent un atome d'hydrogène, de fluor, de chlore ou de brome, un groupe alkyle linéaire ou ramifié en $C_1$ à $C_{12}$, hydroxy, alcoxy linéaire ou ramifié en $C_1$ à $C_{18}$, poly(éthoxy)-alcoxy avec un fragment alkyle en $C_1$ à $C_4$ et le nombre de motif éthoxy compris entre 1 et 4, amino,

ou mono- ou di-alkylamino avec un fragment alkyle en $C_1$ à $C_4$,

- X représente un groupe ortho-, méta- ou para-phénylène, 4,4'-biphénylène, 2,4- ou 2,6- ou 3,4- ou 3,5-pyridinylène, 2,2'-bipyridinylène, méta- ou para-phénylènediamino, éthylènediamino, 2,2'-pipérazinylène, diacyle de formule -$(R_4CO)_2$- où $R_4$ représente un radical phényle, une chaîne alkylée de 3 à 10 atomes de carbone, phénanthrène ou anthracène,
  à l'exception du 1,4-bis(5,6-diphényl-1,2,4-triazin-3-yl)benzène (X=para-phénylène ; $R_1$, $R_2$, $R_3$, $R_4$ = -H), de la 2,4-bis(5,6-diphényl-1,2,4-triazin-3-yl)pyridine, et de la 2,6-bis(5,6-diphényl-1,2,4-triazin-3-yl)pyridine.

Parmi, les composés de formule générale (I), les composés suivants ont conduit à des résultats pratiques tout particulièrement intéressants :

- $R_1$, $R_2$, $R_3$ et $R_4$ représentent un groupe alkyle linéaire ou ramifié en $C_1$ à $C_{12}$ situé en position para, ou un groupe alcoxy linéaire ou ramifié en $C_1$ à $C_{18}$ situé en position para, et
- X représente un groupe 4,4'-biphénylène.

La présente invention a également pour objet les compositions cosmétiques anti-solaires contenant une quantité efficace d'au moins un composé de formule (I) en association avec un excipient cosmétiquement acceptable, de préférence entre 0,1 et 20 % en poids par rapport au poids total de la composition.

Les compositions cosmétiques anti-solaires selon l'invention peuvent contenir en outre un ou plusieurs filtres solaires complémentaires actifs dans l'UV-A et/ou dans l'UV-B et/ou dans l'UV-C (absorbeurs), hydrophiles ou lipophiles. Ces filtres complémentaires peuvent être notamment choisis parmi des dérivés cinnamiques, des dérivés dibenzoylméthane, des dérivés salicyliques, des dérivés du camphre et des dérivés de triazine autres que ceux précédemment cités dans la présente invention.

La présente invention a également pour objet l'utilisation comme filtres solaires actifs dans l'UV-A et/ou l'UV-B et/ou l'UV-C pour la peau humaine et/ou les cheveux, de composés 5,6-diphényl-1,2,4-triaziniques de formule générale (I) :

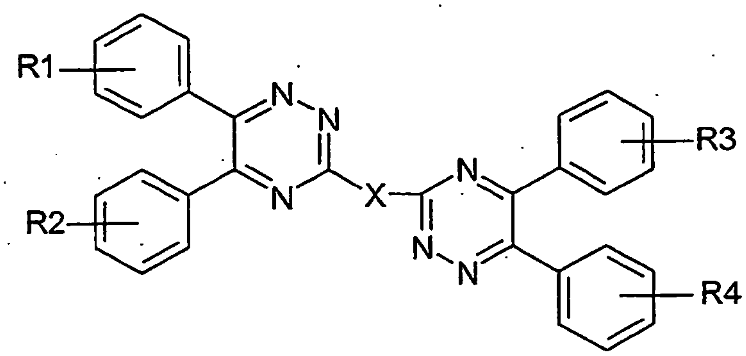

(I)

dans laquelle :

- $R_1$, $R_2$, $R_3$ et $R_4$, identiques ou différents, représentent un atome d'hydrogène, de fluor, de chlore ou de brome, un groupe alkyle linéaire ou ramifié en $C_1$ à $C_{12}$, hydroxy, alcoxy linéaire ou ramifié en $C_1$ à $C_{18}$, poly(éthoxy)-alcoxy avec un fragment alkyle en $C_1$ à $C_4$ et le nombre de motif éthoxy compris entre 1 et 4, amino,

ou mono- ou di-alkylamino avec un fragment alkyle en $C_1$ à $C_4$,

- X représente un groupe ortho-, méta- ou para-phénylène, 4,4'-biphénylène, 2,4- ou 2,6- ou 3,4- ou 3,5-pyridinylène, 2,2'-bipyridinylène, méta- ou para-phénylènediamino, éthylènediamino, 2,2'-pipérazinylène, diacyle de formule -$(R_4CO)_2$- où $R_4$ représente un radical phényle, une chaîne alkylée de 3 à 10 atomes de carbone, phénanthrène ou anthracène.

La présente invention a également pour objet l'utilisation des composés tels que précédemment définis comme agents protecteurs de la lumière actifs dans l'UV-A et/ou l'UV-B et/ou l'UV-C, utiles dans l'industrie des matériaux synthétiques, notamment comme agents protecteurs de la lumière entrant dans la composition de matières plastiques, de verre ou de matières textiles.

Ces composés objets de la présente invention peuvent ainsi être utilisés pour protéger les matériaux photosensibles.

L'agent de protection contre la lumière peut-être incorporé dans un substratum dans le but de protéger ce substratum contre l'attaque des rayons ultraviolets, pour empêcher la modification d'une ou de plusieurs propriétés physiques de ce substratum, comme par exemple la décoloration, une modification de la résistance au déchirage, une augmentation de la fragilité, etc.... et/ou des réactions chimiques provoquées par les rayons ultraviolets, par exemple dans un processus d'oxydation. Dans ce cas, on peut incorporer l'agent de protection avant et pendant la préparation du substratum, ou ultérieurement par un procédé convenable, par exemple un procédé de fixage analogue à une opération de teinture.

L'agent de protection contre la lumière peut aussi être incorporé dans un substratum pour protéger une ou plusieurs autres substances incorporées dans ledit substratum, par exemple des colorants, des agents auxiliaires, etc...

L'agent de protection contre la lumière peut aussi être incorporé dans une couche filtrante pouvant être solide (film, feuille) ou demi-solide (crème, huile, cire), que l'on applique sur un substratum, dans le but de protéger celui-ci des rayons ultraviolets.

Les composés de la présente invention conviennent non seulement comme agents de protection contre la lumière pour des matières incolores, mais également pour des matières pigmentées. Dans ce cas, la protection contre la lumière s'exerce aussi sur les colorants, permettant ainsi dans bien des cas une amélioration tout à fait notable de la stabilité à la lumière.

Les composés de formule générale (I) peuvent être préparés à partir de 1,2-dicétones de formule (II), par des méthodes conventionnelles connues de l'homme de l'art, comme celles décrites dans les exemples qui suivent.

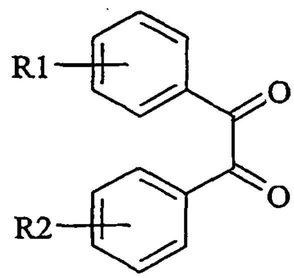

dicétone (II)

où $R_1$ et $R_2$ ont les mêmes significations que celles données précédemment.

Les dicétones de formule (II) sont disponibles dans le commerce (comme par exemple le benzil (diphényléthan-1,2-dione), 4,4'-diméthylbenzil, 4,4'-dibromobenzil, 4,4'-diflurorobenzil ou 4,4'-dichlorobenzil) ou peuvent être synthétisées par des méthodes conventionnelles bien connues de l'homme du métier. Par exemple, on peut utiliser la voie de synthèse suivante :

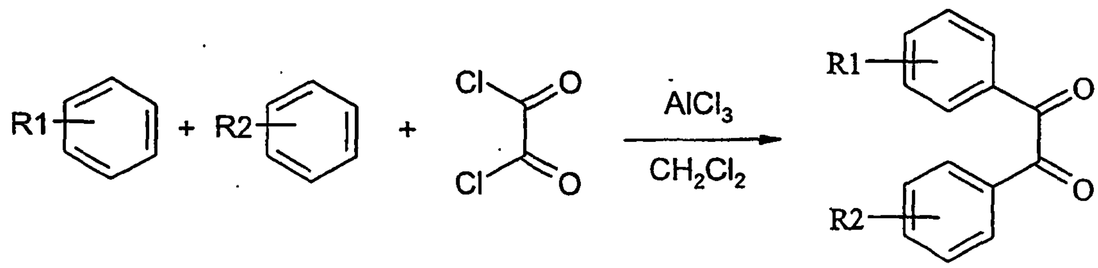


dicétone (II)

Les exemples qui suivent donnent d'autres exemples de synthèses des dicétones de formule (II).

On illustrera ci-après la présente invention en mentionnant quelques exemples non limitatifs de préparation de dérivés représentatifs répondant aux formules générales (I).

Les composés préparés sont rassemblés dans le tableau 1.

**TABLEAU 1**

| |
|---|
| 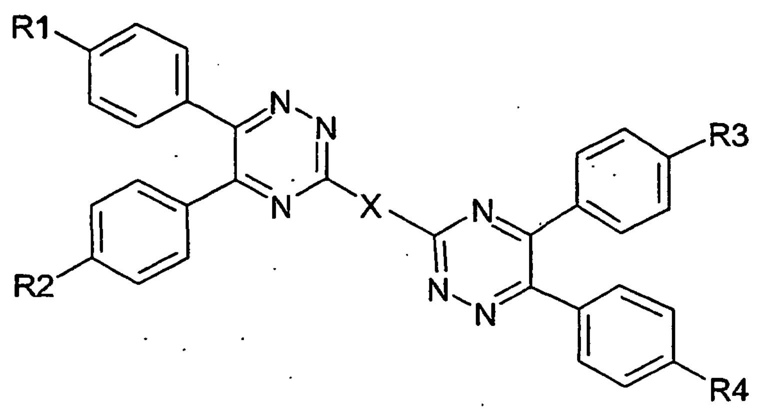 (I) |
| (où R1, R2, R3 et R4 sont en position para) |

(suite)

| Référence | X | $R_1$, $R_2$, $R_3$, $R_4$ |
|---|---|---|
| WP30 | 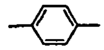 | H |
| WP35 | 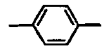 | OH |
| WP38 | 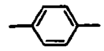 | $Me-(CH_2)_{17}-O-$ |
| WP39 | 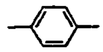 | Me |
| WP40 | 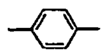 | MeO- |
| WP41 | 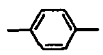 | $MeO-(OCH_2-CH_2)_4-$ |
| WP52 | 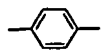 | Br |
| WP89 | 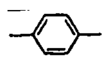 | F |
| WP96 | 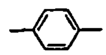 | $Me-(CH_2)_5-$ |
| WP100 | 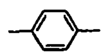 | Cl |
| WP104 | 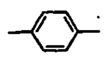 | *tert*-Bu |
| WP107 | 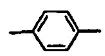 | $Me-(CH_2)_{11}.$ |
| WP144 | 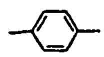 | $Me-(CH_2)_3-CH(Et)-CH_2-O-$ |
| WP149 | 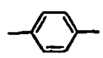 | $Me-(CH_2)_5-CH(Me)-O-$ |
| WP151 | 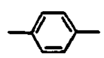 | $Me-CH(Me)-(CH_2)_2-CH(Me)_2-$ |
| WP135 | 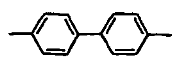 | -H |
| WP76 | 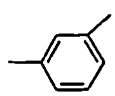 | -H |

Les composés de formule (I) du tableau 1 peuvent être synthétisés de la façon suivante :

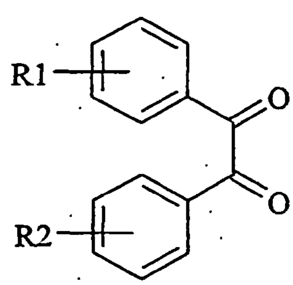

dicétone (II)

où R1, R2 = hydrogène, halogène
-OH,
alcoxy linéaire ou ramifié en $C_1$ à $C_{18}$ (tel que -OMe),
alkyle linéaire ou ramifié en $C_1$ à $C_{12}$,
mono ou dialkylamino avec un fragment alkyle en $C_1$ à $C_4$ (tel que $N(Et)_2$).

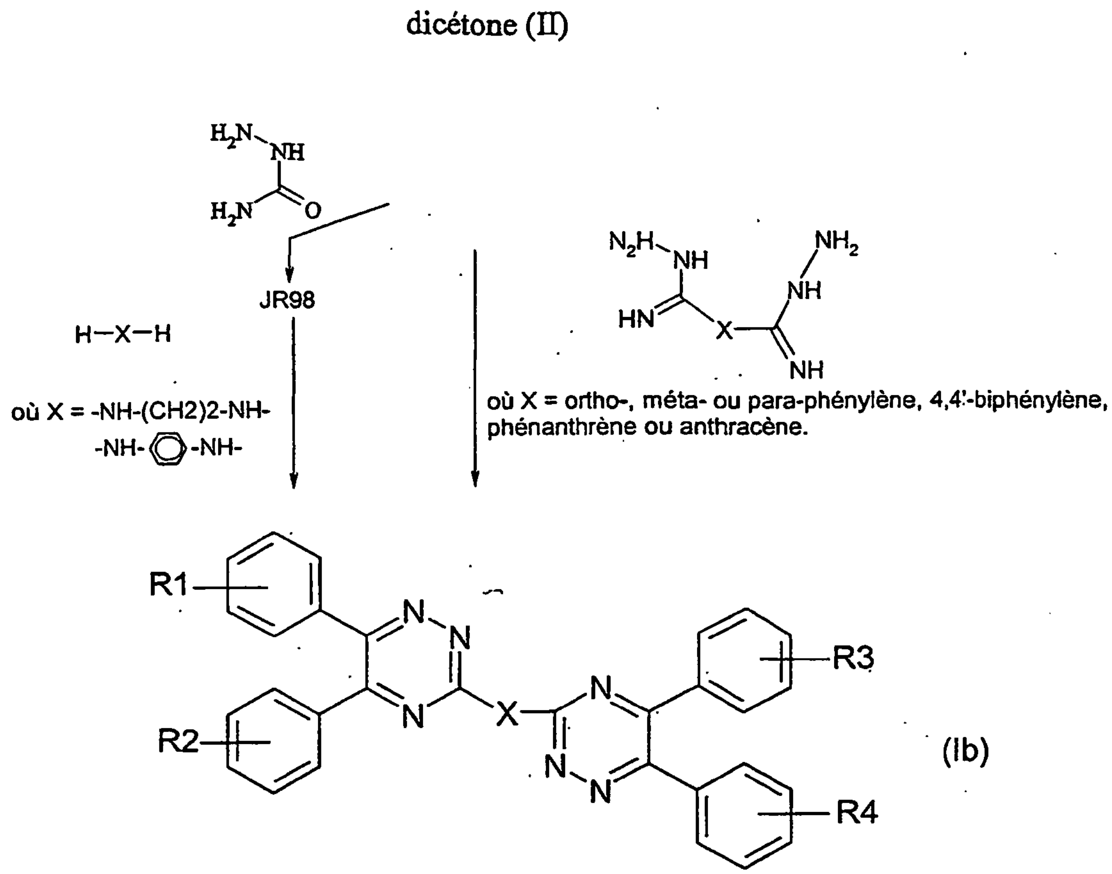


(I)

**FIGURES:**

DBM = Parsol 1789® (Laboratoires ROCHE)
MCX = Parsol MCX® (Laboratoires ROCHE)

La **figure 1** représente le spectre d'absorption dans l'UVA et l'UVB des composés WP89, WP96, WP100, WP104, WP107, WP135, WP144, WP149. et WP151.
La **figure 2** représente le spectre d'absorption dans l'UVA et l'UVB des composés WP35, WP39, WP41, WP52 et WP30.
La **figure 3** représente le spectre d'absorption dans l'UVA et l'UVB du composé WP76.

**Exemple 1 : Synthèse de la 1,2-bis(4-méthoxyphényl)-éthane-1,2-dione[(1)]**

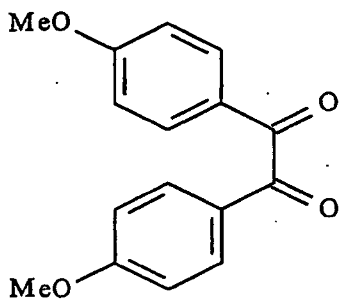

$C_{16}H_{14}O_4$ M=270,28 g.mol$^{-1}$

A un mélange d'anisole (10,8 g, 100 mmol) et de chlorure d'aluminium (33,33 g, 250 mmol) est ajouté lentement du chlorure d'oxalyle (4,71 ml, 55,2 mmol) à 0˚C. Le mélange est agité à température ambiante pendant 4 heures. Après refroidissement, il est jeté dans de l'eau glacée et extrait au dichlorométhane. Les phases organiques rassemblées sont lavées par HCl 2N puis par de la saumure et séchées sur sulfate de magnésium. Après filtration et concentration sous pression réduite, le résidu est recristallisé dans de méthanol. Le précipité résultant est filtré, lavé plusieurs fois à méthanol et séché pour donner 9,80 g (66%) de produit pur sous la forme d'un solide jaune. $^{\delta}$H (200 MHz, $CDCl_3$) 3,93 (s ; 6H), 6,99 (d; *J* 7,8; 4H), 7,99 (d; *J* 7,8; 4H).

**Exemple 2 : Synthèse du Téréphthalimidate de diéthyle[(2)] (WP18)**

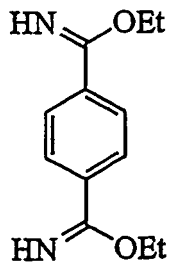

$C_{12}H_{16}N_2O_2$ M = 220,27 g.mol$^{-1}$

A une suspension de téréphthalonitrile (12,81 g; 100 mmol) dans de l'éthanol absolu (250 ml) refroidie à 0 ˚C est effectué un bullage de HCl gazeux pendant 18 heures, durant lesquelles la température est remontée à l'ambiante. Le solide blanc obtenu (27,5 g de sel di-chlorohydrate) est alors filtré et lavé à l'éthanol. La neutralisation de ce sel dissout dans un minimum d'eau à 0 ˚C est effectuée en ajoutant une solution de potasse (15% aqueux) jusqu'à pH basique. Un solide blanc est obtenu (rendement > 90%) après filtration, lavages successifs à l'eau puis au pentane et séchage. mp 158-161 ˚C; $\delta_H$ (300 MHz, $CDCl_3$) 1,43 (t; *J* 7,2; 6H), 4.32 (q; *J* 7,2; 4H), 7.79 (s, 4H); SM (Electrospray) *m/z* 222 (40%), 221 ($MH^+$, 100%), 193 (M-CH=$CH_2$, 42 %).

**Exemple 3 : Synthèse de la Téréphthalamidrazone[(2)] (WP29)**

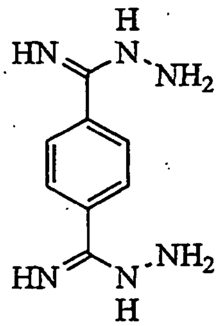

$C_8H_{12}N_6$ $M = 192,2224\ g.mol^{-1}$

A une suspension de WP18 préparé selon l'exemple 2 (9,92 g; 45,035 mmol) dans de l'éthanol absolu (75 mL) est ajoutée de l'hydrazine monohydratée (6,55 mL; 135 mmol) durant 10 minutes. Le milieu devient rapidement homogène, puis un précipité jaune se forme lentement. Après 24 heures, le précipité est filtré, lavé sucessivement à l'éthanol puis à l'éther diéthylique et séché pour donner 6,838 g (79%) d'un solide jaune. $\delta_H$ (300 MHz, $D_2O$) 7,66 (s; 4H).

**Exemple 4 : Synthèse du 1,4-Bis(5,6-diphényl-1,2,4-triazin-3-yl)benzène[3] (WP30)**

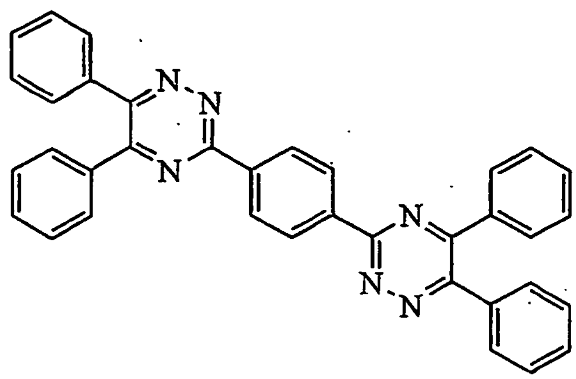

$C_{36}H_{24}N_6$ $M = 540,6258\ g.mol^{-1}$

A une solution de benzil (2,455 g, 11,67 mmol) dans méthanol (100 mL) est ajoutée la téréphthalamidrazone WP 29 préparé selon l'exemple 3 (1,00 g, 5,203. mmol) en une fois. Le milieu est chauffé à reflux pendant 15 heures: Après retour à température ambiante, le précipité obtenu est filtré, lavé successivement à l'éthanol puis à l'éther diéthylique et séché pour donner 2,587 g (92%) d'un solide jaune. mp = 321 °C; $\delta_H$ (300 MHz, DMSO-*d*, 120 °C) 7,42-7,52 (m; 12H), 7,62 (m; 4H), 7,71 (m; 4H), 8.81 (s; 4H); $\delta_c$ (75 MHz, DMSO-*d*, 120 °C) 129,2 (1), 129,3 (1), 130,2 (1), 130,3 (1), 130,5 (1), 131,4 (1), 136,5 (0), 136,6 (0), 138,4 (0), 156,5 (0), 156,9 (0), 161,3 (0); MS (Nanospray) *m*/*z* 1081 ($2M+H^+$, 22%), 541 ($MH^+$, 41%).

**Exemple 5 : Synthèse du 4,4.'-Dihydroxybenzil (4) (WP32)**

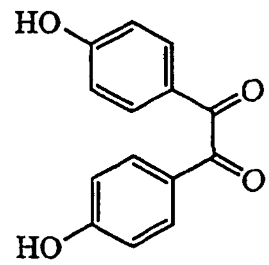

$C_{14}H_{10}O_4$ M = 242,2306 g.mol$^{-1}$

Un mélange de 1,2-bis(4-méthoxyphényl)-éthane-1,2-dione préparée selon l'exemple 1 (7,275 g; 26,91 mmol) et de chlorhydrate de pyridine (15,55 g, 134,5 mmol) sous atmosphère d'azote est chauffé à 180 °C pendant 2 jours. Après retour à température ambiante, le milieu est dilué par de l'acétate d'éthyle. et de l'eau. La phase aqueuse est extraite à l'acétate d'éthyle et les phases organiques réunies sont séchées sur sulfate de magnésium, filtrées et concentrées sous pression réduite. Le résidu est purifié par flash chromatographie sur gel de silice (éther de pétrole/acétate d'éthyle 10/1 à 2/1) pour donner 5,789 g (89%) d'un solide blanc. $\delta_H$ (300 MHz, MeOH-d) 6,91 (d; *J 9, 0;* 4H), 7,82 (d; *J 9, 0;* 4H).

**Exemple 6 : p-Tosylate de 2-{2-[2-(2-méthoxy-éthoxy)-éthoxy]-éthoxy}-éthyle[(5)] (WP33)**

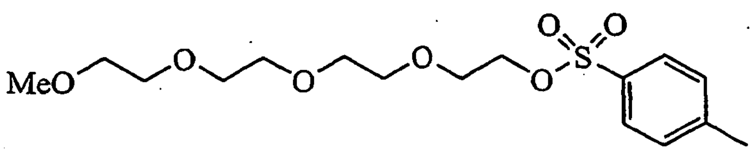

$C_{16}H_{26}O_7S$ M = 362,4432 g.mol$^{-1}$

A un mélange de tétraéthylèneglycol monométhyl éther (3,00 g, 14,406 mmol) et de soude (0,865 g, 21,6 mmol) dilué dans du THF (33 mL) et de l'eau (4 mL), refroidi à 0°C, est ajoutée lentement une solution de chlorure d'acide p-toluènesulfonique (3,021 g, 15,8 mmol) dans le THF (4 mL). Après 3 heures d'agitation à 0°C, le milieu est jeté dans de l'eau glacée (10 mL) et dilué par du dichlorométhane. La phase aqueuse est extraite au dichlorométhane et les phases organiques réunies sont lavées à l'eau puis par une solution saturée de NaCl, séchées' sur $MgSO_4$, filtrées et concentrées sous pression réduite. Le résidu est purifié par flash chromatographie sur gel de silice (éther de pétrole/acétate d'éthyle 1/1 à 1/4) pour donner 4,234 g (82%) d'une huile incolore. $\delta_H$ (300 MHz, $CDCl_3$) identique à la littérature.

**Exemple 7 : Synthèse du 1.4-Bis[5.6-(4,4'-dihydroxy)-diphényl-1,2,4-triazin-3-yl]benzène (WP35)**

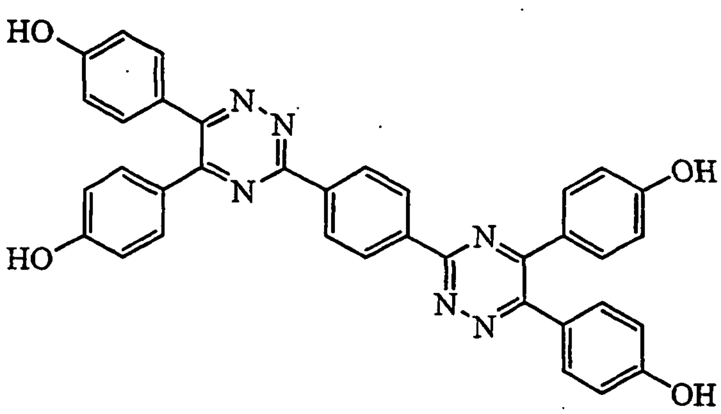

$C_{36}H_{24}N_6O_4$ M = 604,6228 g.mol$^{-1}$

A une solution de WP32 préparée selon l'exemple 5 (600 mg, 2,47 mmol) dans l'éthanol (20 mL) est ajoutée la téréphthalamidrazone WP 29 préparée selon l'exemple 3 (190 mg, 0,991 mmol) en une fois. Le milieu est chauffé à reflux pendant 15 heures. Après retour à température ambiante, le précipité obtenu est filtré, lavé successivement à l'éthanol puis à l'éther diéthylique et séché pour donner 479 mg (80%) d'un solide jaune. $\delta_H$ (300 MHz, DMSO-*d*, 25 °C) 6,79-6,84 (m; 8H), 7,44 (d; *J 8,4;* 4H), 7,58 (d; *J 8,4;* 4H), 8.71 (s; 4H), 10,03 (sl, 4H); MS (Nanospray) *m/z* 605 ($MH^+$, 53 %)

**Exemple 8: Synthèse de la 1,2-Bis-[4-(2-{2-[2-(2-méthoxy-éthoxy)-éthoxy]-éthoxy}-éthoxy)-phényl]-éthane-1,2-dione (WP36)**

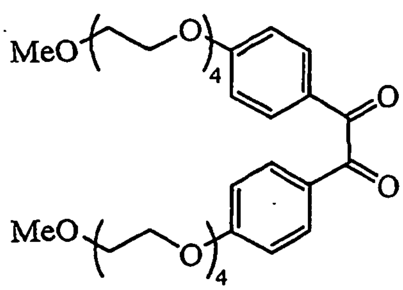

$C_{32}H_{46}O_{12}$ M = 622,7082 g.mol$^{-1}$

A une solution du tosylate WP33 préparé selon l'exemple 6 (3,737 g, 10,3 mmol) dans le DMF (50 mL) sont successivement ajoutés le 1,2-bis(4-méthoxyphényl)-éthane-1,2-dione préparée selon l'exemple 1 (1,135 g, 4,687 mmol) puis du carbonate de potassium (3,239 g, 23,4 mmol). Le mélange est agité à 50 °C pendant 4 heures. Après retour à température ambiante, le milieu est jeté dans de l'eau glacée et extrait plusieurs fois par de l'acétate d'éthyle. Les phases organiques réunies sont lavées plusieurs fois par une solution saturée de $NaHCO_3$, puis par de la saumure. Après séchage sur $MgSO_4$, fitration et concentration sous pression réduite, le résidu obtenu est purifié par flash chromatographie sur gel de silice (acétate d'éthyle, puis dichlorométhane/méthanol 1% à 1,5%) pour donner 1,568 g (54%, non optimisé) d'une huile jaune. $\delta_H$ (300 MHz, $CDCl_3$) 3,36 (s; 6H), 3,51 (m; 4H), 3,5 1-3,71 (m; 20H), 3,87 (m, 4H), 4,19 (m, 4H), 6,97 (d, *J* 8,8; 4H), 7,92 (d, *J* 8,8; 4H). MS (Electrospray) *m/z* 623 ($MH^+$, 100%).

**Exemple 9 : Synthèse de la 1,2-Bis-(4-octadécyloxy-phényl)-éthane-1,2-dione(6) (WP37)**

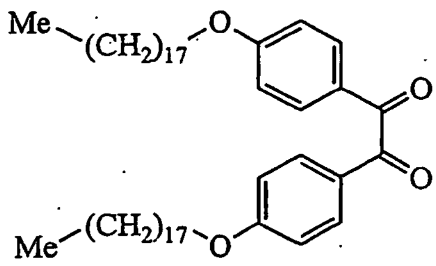

$C_{50}H_{82}O_4$ M = 747,1954 g.mol$^{-1}$

A une solution de 1,2-bis(4-méthoxyphényl)-éthane-1,2-dione préparée selon l'exemple 1 (2,00 g; 8,25 mmol) dans le DMF (50 mL) est ajoutée de la soude (726 mg; 18,1 mmol). Le mélange est agité 5 minutes à température ambiante, puis du 1-iodooctadécane (9,4 g; 24,7 mmol) est ajouté en 1 minute. Après 3 heures d'agitation à 60 °C, le milieu est refroidi. Le précipité obtenu est filtré, lavé successivement au DMF puis à l'éthanol et séché sous vide pour donner 3,792 g (61%) d'un solide blanc. mp 87 °C; $\delta_H$ (300 MHz, $C_6D_6$) 0,90 (t; *J 6,0;* 6H), 1,18-1,42 (m; 60H), 1,51 (m, 4H), 3,46 (t; *J* 6, 5; 4H), 6,64 (d; *J 9, 0;* 4H), 8,09 (d; *J 9, 0;* 4H); MS (Electrospray) *m/z* 1493 (2M+H$^+$, 5%), 747 (MH$^+$, 26%).

**Exemple 10: Synthèse du 1,4-Bis[5,6-(4,4'-di-octadécyloxy)-diphényl-1,2,4-triazin-3-yl] benzène (WP38)**

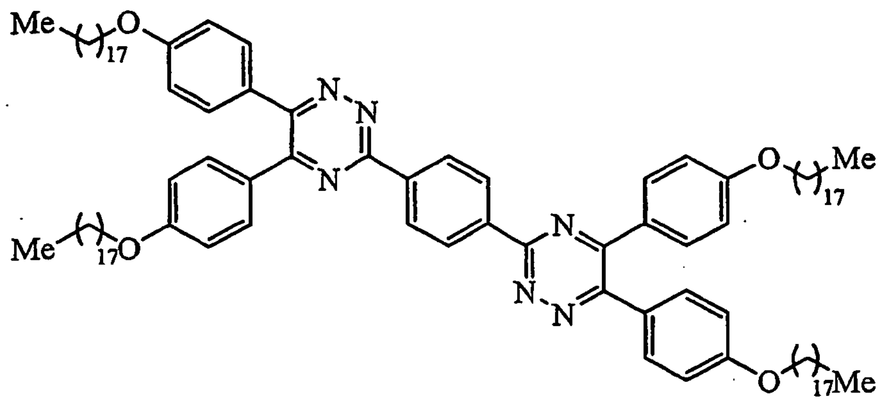

$C_{108}H_{168}N_6O_4$ M = 1614,553 g.mol$^{-1}$

A une solution de WP37 préparé selon l'exemple 9 (1,71 g, 22,88 mmol) dans l'éthanol (20 mL) est ajoutée la téréphthalamidrazone WP 29 préparée selon l'exemple 3 (200 mg, 1,04 mmol) en une fois. Le milieu est chauffé à reflux pendant 15 heures. Après retour à température ambiante, le précipité obtenu est filtré et lavé à l'éthanol, puis purifié par flash chromatographie sur gel de silice (toluène, puis toluène/méthanol 1% à 2%) pour donner 1,409 g (84%) d'un solide jaune. $\delta_H$ (300 MHz, $C_6D_6$) 0,91 (m; 6H), 1,18-1,42 (m; 120H), 1,51 (m, 8H), 3,60 (m; 8H), 6,75 (d; *J 9, 0;* 4H), 6,81 (d; *J 9, 0;* 4H), 7,71 (d; *J 9, 0;* 4H), 7,73 (d; *J 9, 0;* 4H), 9,22 (s, 4H); MS (Electrospray) *m/z* 1615 (MH$^+$, 64%), 1614 (75%), 890 (100%).

**Exemple 11 : Synthèse du 1,4-Bis[5,6-(4,4'-diméthyl)-diphényl-1,2,4-triazin-3-yl]benzène (WP39)**

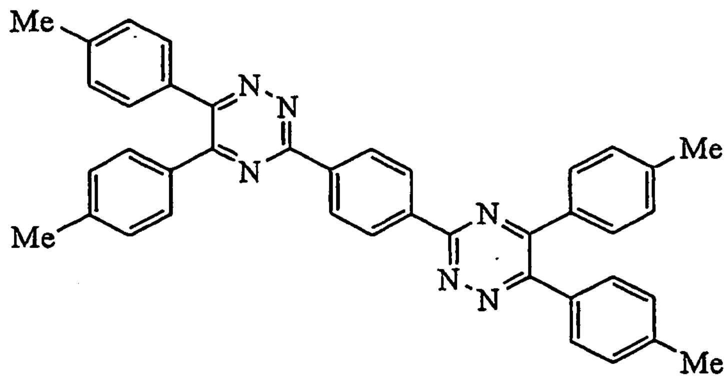

$C_{40}H_{32}N_6$ $M = 596,733\ g.mol^{-1}$

A une solution de 4,4'-diméthylbenzil (4,091 g, 17,16 mmol) dans l'éthanol (140 mL) est ajoutée la téréphthalamidrazone WP 29 préparée selon l'exemple 3 (1,50 g, 7,804 mmol) en une fois. Le milieu est chauffé à reflux pendant 15 heures: Après retour à température ambiante, le précipité obtenu est filtré, lavé successivement à l'éthanol puis à l'éther diéthylique et séché pour donner 4,42 g (95%) d'un solide jaune. $\delta_H$ (300 MHz, DMSO-*d*, 120 °C) 2,50 (s, 12H), 7,27 (m; 8H), 7,52 (d; *J* 8, 0; 4H), 7,61 (d; *J 8, 0;* 4H), 8,78 (s; 4H); MS (Nanospray) *m/z* 1789 (3M+H$^+$, 65%), 1193 (2M+H$^+$, 57%), 597 (MH$^+$, 49%).

**Exemple 12 : Synthèse du 1,4-Bis[5,6-(4,4'-diméthoxy)-diphényl-1,2,4-triazin-3-yl]benzène (WP40)**

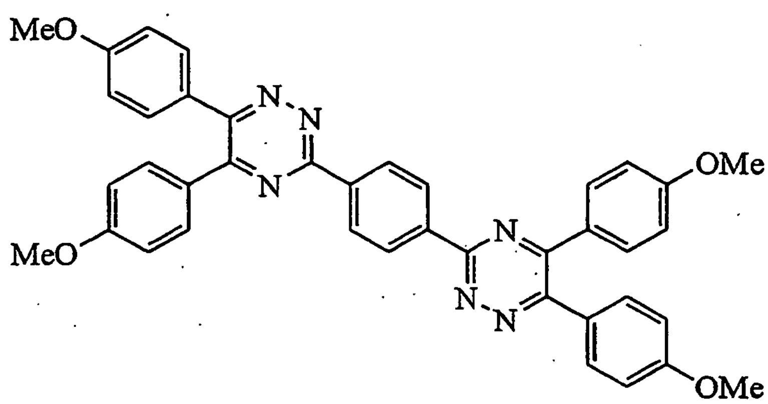

$C_{40}H_{32}N_6O_4$ $M = 660,7306\ g.mol^{-1}$

A une solution de 1,2-bis(4-méthoxyphényl)-éthane-1,2-dione préparée selon l'exemple 1 (5,273 g, 19,5 mmol) dans l'éthanol (140 mL) est ajoutée la téréphthalamidrazone WP 29 préparée selon l'exemple 3 (1,50 g, 7,804 mmol) en une fois. Le milieu est chauffé à reflux pendant 30 heures. Après retour à température ambiante, le précipité obtenu est filtré, lavé successivement à l'éthanol, au dichlorométhane et à l'éther diéthylique puis séché pour donner 4,087 g (80%) d'un solide jaune. $\delta_H$ (300 MHz, DMSO-*d*, 120 °C) 3,86 (s, 12H), 7,02 (m; 8H), 7,58 (d; *J* 7, 8; 4H), 7,71 (d; *J* 8, 7; 4H), 8,77 (s; 4H); MS (Nanospray) *m/z* 661 (MH$^+$, 8%), 332 (55%).

**Exemple 13 : Synthèse du 1,4-Bis[5,6-(4,4'-(2-{2-[2-(2-méthoxy-éthoxy)-éthoxy]-éthoxy}-éthoxy)-)-diphényl-1,2,4-triazin-3-yl]bénzène (WP41)**

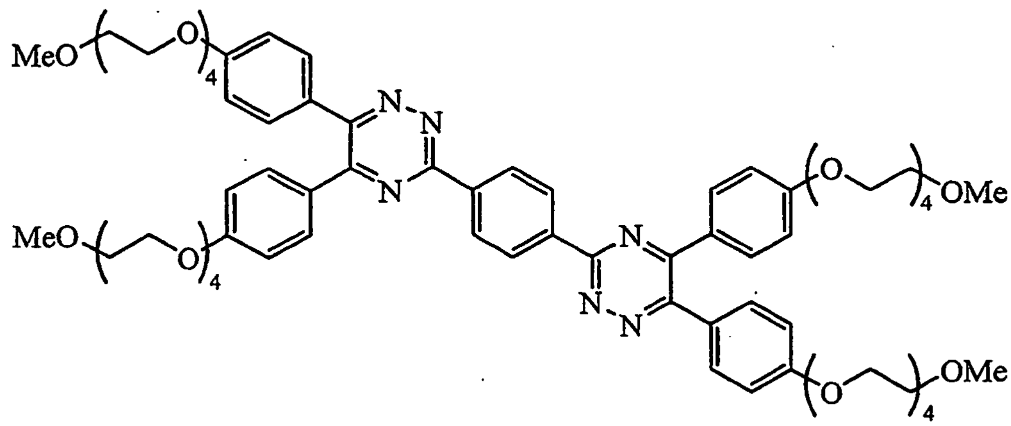

$C_{72}H_{96}N_6O_{20}$ M = 1365,5786 g.mol$^{-1}$

A une solutions de WP36 préparé selon l'exemple 8 (524 mg; 0,842 mmol) dans l'éthanol (7 mL) est ajoutée la téréphthalamidrazone WP 29 préparée selon l'exemple 3 (64 mg; 0,337 mmol) en une fois. Le milieu est chauffé à reflux pendant 30 heures. Après retour à température ambiante, le solvant est concentré sous pression réduite et le résidu est directement purifié par flash chromatographie sur gel de silice (acétate d'éthyle, puis acétate d'éthyle/méthanol 2% à 6%) pour donner 350 mg (76%) d'un solide jaune. $\delta_H$ (300 MHz, $CDCl_3$) 3,37 (s; 12H), 3,54 (m; 8H), 3,60-3,74 (m; 40H), 3,88 (m, 8H), 4,18 (m, 8H), 6,93 (m, 8H), 7,60 (d, *J 8,8;* 4H), 7,92 (d, *J* 8,8; 4H);8,82 (s; 4H); $\delta_c$ (75 MHz, $CDCl_3$) 58,9 (3), 67,3 (2), 67,4 (2), 69,4 (2), 69,5 (2), 70,4 (2), 70,5 (2), 70,7 (2), 71,8 (2), 114,5 (1), 114,6 (1), 128,0 (1), 128,1 (1), 128,4 (1), 130,6 (1), 131,4 (1), 137,5 (1), 154,2 (0), 154,7 (0), 159,9 (0), 160,1 (0), 160,9 (0) ; MS (Electrospray) *m/z* 1387 (M+Na$^+$, 24%), 1366 (MH$^+$, 100%).

**Exemple 14: Synthèse du 1,4-Bis-[5,6-(4,4'-dibromo)-diphényl-1,2,4-triazin-3-yl]benzène (WP52)**

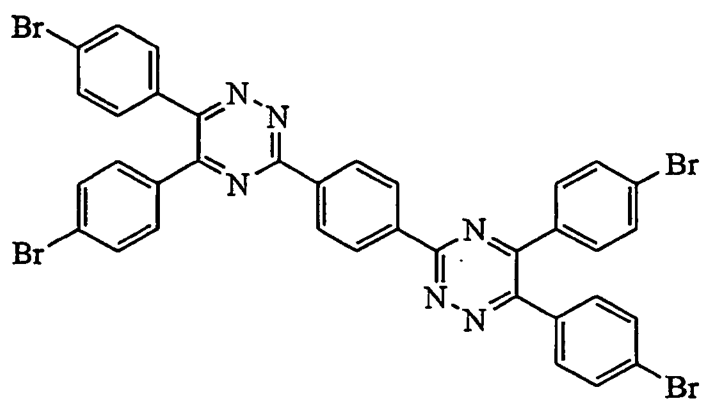

$C_{36}H_{20}Br_4N_6$ M = 856,2102 g.mol$^{-1}$

A une solution de 4,4'-dibromobenzil (1,149 g, 3,12 mmol) dans l'éthanol (20 mL) est ajoutée la téréphthalamidrazone WP 29 préparée selon l'exemple 3 (200 mg, 1,040 mmol) en une fois. Le milieu est chauffé à reflux pendant 15 heures. Après retour à température ambiante, le précipité obtenu est filtré, lavé successivement à l'éthanol, au dichlorométhane et à l'éther diéthylique puis séché pour donner 757 mg (85%) d'un solide jaune. $\delta_H$ (300 MHz, DMSO-*d*, 120 ˚C) 7,56-7,69 (m; 8H), 7,80-7,88 (m; 8H), 8,80 (s; 4H).

**Exemple 15 : Synthèse du 4-Bromododécanoylbenzène[7] (WP59)**

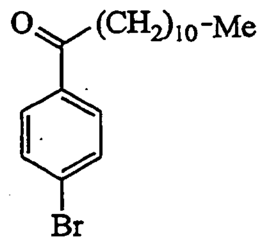

$C_{18}H_{27}BrO$ $M = 339.3147\ g.mol^{-1}$

A un mélange de bromobenzène (60 g, 382.1 mmol) et de chlorure d'aluminium (30.57 g, 229.3 mmol) est ajouté lentement du chlorure de dodécanoyle (44.1 mL, 191.1 mmol). Le mélange est agité à 50 °C pendant une heure. Après refroidissement, il est jeté dans de l'eau glacée et extrait au dichlorométhane. Les phases organiques rassemblées sont lavées par HCl 2N puis par de la saumure et séchées sur sulfate de magnésium. Après filtration et concentration sous pression réduite, le résidu est repris dans de l'éthanol. Le précipité résultant est filtré, lavé plusieurs fois à l'éthanol et séché pour donner 36.9 g (57%, non optimisé) de produit pur sous la forme d'un solide blanc (pas de recristallisation). Analyses identiques à la littérature.

**Exemple 16 : Synthèse du 4-Bromododécylbenzène[7] (WP60),**

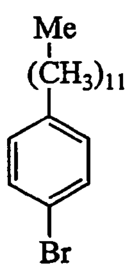

$C_{18}H_{29}Br$ $M = 325.3311\ g.mol^{-1}$

A une solution de WP59 préparé selon l'exemple 15 (36.5 g, 108.87 mmol) dans le tri(éthylène glycol) (180 mL) est ajoutée de l'hydrazine monohydratée (23.6 mL, 4.5 éq.) puis de la potasse (24.3 g, 4 éq.). Le mélange est agité à reflux pendant environ 15 heures. Après refroidissement, il est jeté dans de l'eau, acidifié avec HCl concentré, puis extrait au dichlorométhane. La phase organique est lavée par de l'eau, séchée sur sulfate de magnésium, filtrée et concentrée sous pression réduite. Le résidu est purifié par flash chromatographie sur gel de silice (pentane 100%) pour donner 21,2 g (60%, non optimisé) d'une huile incolore. Analyses identiques à la littérature.

**Exemple 17 : Isophthalimidate de diéthyle (WP73)**

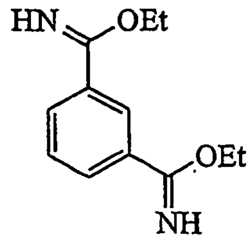

$C_{12}H_{16}N_2O_2$ $M = 220,27\ g.mol^{-1}$

A une suspension d'isophthalonitrile (12,81 g; 100 mmol) dans un mélange 1,4-dioxanne sec (100 ml)/éthanol absolu (14,6 mL) refroidie à 0 °C est effectué un bullage de HCl gazeux pendant 48 heures, durant lesquelles la température est remontée à l'ambiante. Après 4 jours d'agitation supplémentaires, le solide blanc obtenu (environ 28 grammes de sel di-chlorohydrate) est filtré et lavé à l'éther diéthylique. La neutralisation de ce sel mis en suspension dans de l'éther diéthylique est effectuée en ajoutant lentement une solution aqueuse de carbonate de potassium (30% en poids) jusqu'à pH basique. La phase organique est séparée, séchée sur $MgSO_4$, filtrée et concentrée sous pression réduite pour donner un solide blanc (rendement > 90%). $\delta_H$ (300 MHz, $CDCl_3$) 1,44 (t; *J* 6,9; 6H), 4.32 (q; J 6,9; 4H), 7,47 (t; J 7,5; 1H), 7,86 (d; *J* 7,5; 2H), 8,16 (s, 1H); SM (Electrospray) *m*/*z* 221 ($MH^+$, 100%).

**Exemple 18 : Isophthalamidrazone[(8)] (WP75)**

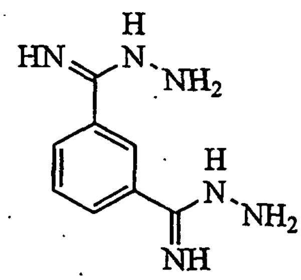

$C_8H_{12}N_6$ $M = 192,2224\ g.mol^{-1}$

A une suspension d'isophthalimidate de diéthyle WP 73 préparée selon l'exemple 17 (1,0 g; 4,53 mmol) dans de l'acétonitrile sec (18 mL) refroidie à 0 °C est. ajoutée de l'hydrazine monohydratée (485 μL; 9,98 mmol) durant 10 minutes. Après 48 heures d'agitation, le précipité formé est filtré, lavé à l'acétonitrile et séché pour donner 630 mg (72%) d'un solide jaune. $\delta_H$ (300 MHz, $D_2O$) 7,50 (t; *J*7,3; 1H), 7,67 (d; J7,3; 2H), 7,80 (s, 1H); SM (en solution dans $D_2O$) (EI) *m*/*z* 199 (100%).

**Exemple 19 : 1,3-Bis(5,6-diphényl-1,2,4-triazin-3-yl)benzène (WP76)**

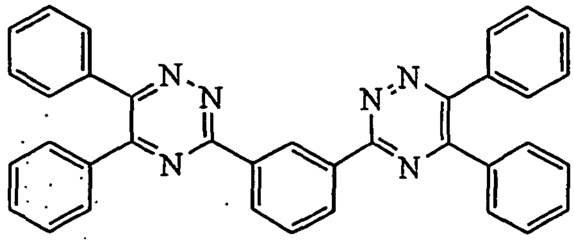

$C_{36}H_{24}N_6$ M = 540,6258 g.mol$^{-1}$

A une solution de benzil (1,491 g, 7,09 mmol) dans l'éthanol (60 mL) est ajouté WP75 préparé selon l'exemple 18 (620 mg, 3,225 mmol) en une fois. Le milieu est chauffé à reflux pendant 20 heures. Après retour à température ambiante, le précipité obtenu est filtré, lavé successivement à l'éthanol puis à l'éther diéthylique et séché pour donner 1,488 g (85%) d'un solide jaune. $\delta_H$ (300 MHz, DMSO-*d*, 120 °C) 7,42-7,52 (m; 12H), 7,61 (d; *J 1,2;* 4H), 7,63 (d; *J 1,5;* 4H), 7,90 (t; *J* 7,3; 1H), 8,81 (d; *J* 7,3; 2H), 9,77 (s; 1H); MS (Electrospray) *m/z* 1081 (2M+H$^+$, 74%), 541 (MH$^+$, 100%), 175 (88%).

**Exemple 20 : Synthèse du 1,4-Bis[5,6-(4,4'-difluoro)-diphényl-1,2,4-triazin-3-yl]benzène (WP89)**

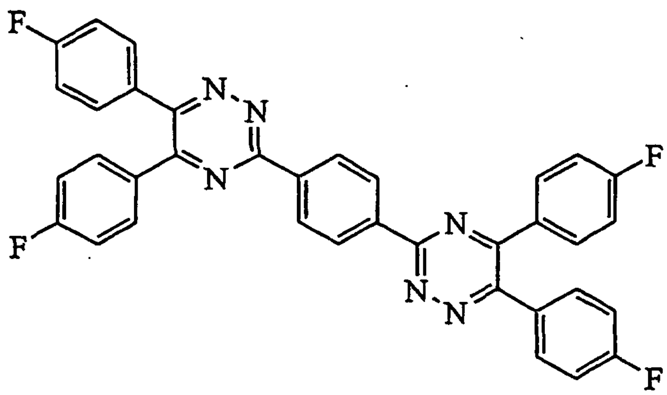

$C_{36}H_{20}F_4N_6$ M = 612,5878 g.mol$^{-1}$

A une solution de 4,4'-difluorobenzil (769 mg, 3,12 mmol) dans l'éthanol (20 mL) est ajoutée la téréphthalamidrazone WP 29 préparée selon l'exemple 3 (200 mg, 1,040 mmol) en une fois. Le milieu est chauffé à reflux pendant 15 heures. Après retour à température ambiante, le précipité obtenu est filtré, lavé successivement à l'éthanol puis à l'éther diéthylique et séché sous vide pour donner 541 mg (85%) d'un solide jaune. $\delta_H$ (300 MHz, DMSO-*d*, 120 °C) 7,56-7,69 (m; 8H), 7,80-7,88 (m; 8H), 8,80 (s; 4H). MS (Electrospray) *m/z* 613 (MH$^+$, 100%), 178 (34%).

**Exemple 21 : Synthèse de la 1,2-Bis-(4-hexylphényl)-éthane-1,2-dione (WP94)**

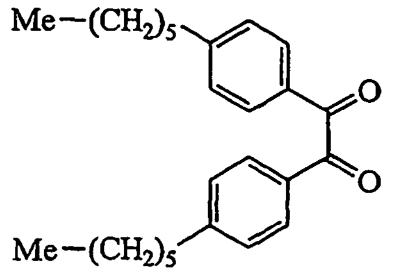

$C_{26}H_{34}O_2$ $M = 378.5534\ g.mol^{-1}$

Une solution de s-BuLi (1.3 M in cyclohexane, 5.4 mL, 7.02 mmol) est ajoutée lentement à une solution de 4-bromo-n-hexylbenzène (1.68 g, 7.02 mmol) dans le THF (9 mL) à -78 °C et sous atmosphère d'azote. Après une heure d'agitation, le mélange est transféré à l'aide d'une canule à une suspension de 1,4-diméthylpipérazi.ne-2,3-dione (450 mg, 3.166 mmol) dans le THF (11 mL) refroidi à -40 °C. Après retour à température ambiante, le milieu est agité pendant 15 heures puis traité par 5 mL de HCl 2N. Après dilution au dichlorométhane et agitation, la phase organique est séparée, lavée par HCl 2N puis par de l'eau, et séchée sur sulfate de magnésium. Après filtration et concentration, le résidu est purifié par flash chromatographie sur gel de silice (pentane/acétate d'éthyle 100/0; 4/1; 3/1; 2/1) pour donner 820 mg (68%) d'une huile jaune. $\delta_H$ (300 MHz, $CDCl_3$) 0,87 (t; *J* 6,6; 6H), 1,20-1,40 (m; 12H), 1,50-1.70 (m, 4H), 2,67 (t; *J* 7,5; 4H), 7,30 (d; J 8,4; 4H), 7,87 (d; *J 8, 4;* 4H); MS (IC) *m/z* 379 ($MH^+$, 100%).

**Exemple 22: Synthèse du 1,4-Bis[5,6-(4,4'-dihexyl)-diphényl-1,2,4-triazin-3-yl]benzène (WP96)**

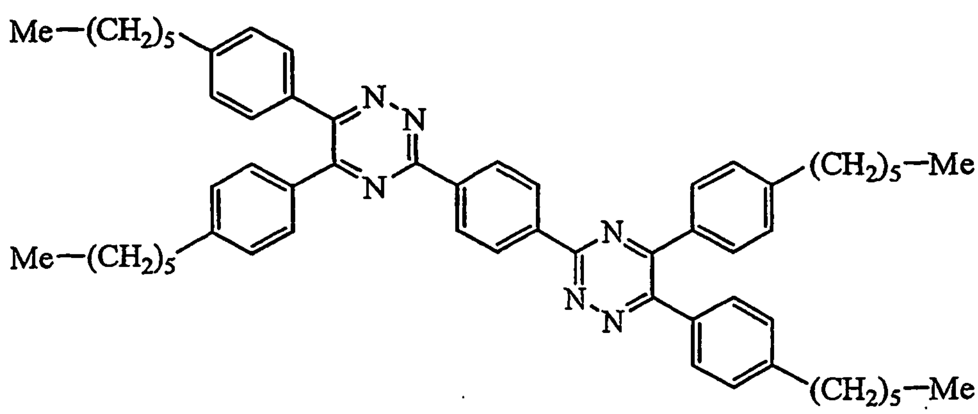

$C_{60}H_{72}N_6$ $M = 877.2690\ g.mol^{-1}$

A une solution de WP94 préparé selon l'exemple 21 (187 mg, 0,49 mmol) dans l'éthanol (5 mL) est ajoutée la téréphthalamidrazone WP 29 préparée selon l'exemple 3 (43 mg, 0.22 mmol) en une fois. Le milieu est chauffé à reflux pendant 15 heures. Après retour à température ambiante, le précipité obtenu est filtré, lavé à l'éthanol et séché pour donner 144 mg (74%) d'un solide jaune. $\delta_H$ (300 MHz, $CDCl_3$) 0,88 (m; 12H), 1,20-1,40 (m; 24H), 1,45-1.70 (m, 8H), 2,65 (t; *J* 7,8; 8H), 7,18 (m; 8H), 7,57 (d; *J 8,4;* 4H), 7,65 (d; *J* 7,8; 4H); MS (Electrospray) *m/z* 1754 ($2M+H^+$, 33%), 877 ($MH^+$, 100%).

**Exemple 23 : Synthèse du 1,4-Bis[5,6-(4,4'-dichloro)-diphényl-1,2,4-triazin-3-yl]benzène (WP100)**

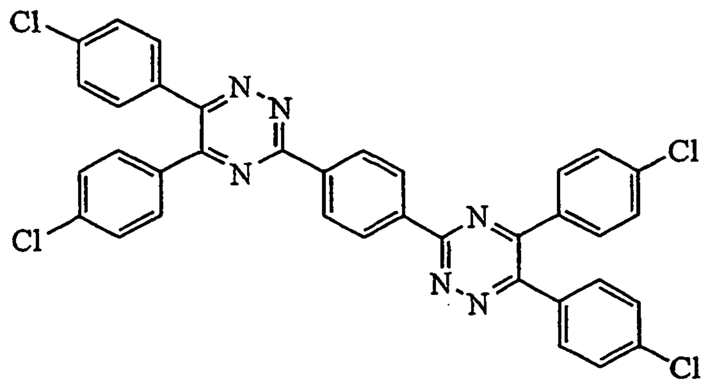

$C_{36}H_{20}Cl_4N_6$ M = 678,405 $g.mol^{-1}$

A une solution de 4,4'-chlorobenzil (75 mg, 0,27 mmol) dans l'éthanol (3 mL) est ajoutée la téréphthalamidrazone WP 29 préparée selon l'exemple 3 (24 mg, 0,122 mmol) en une fois. Le milieu est chauffé à reflux pendant 15 heures. Après retour à température ambiante, le précipité obtenu est filtré, lavé successivement à l'éthanol puis à l'éther diéthylique et séché sous vide pour donner un solide jaune (rendement non calculé). $\delta_H$ (300 MHz, DMSO-*d*, 120 °C) 7,50-7,60 (m; 12H), 7,69 (d; *J* 8, 4; 4H), 8,81 (s; 4H).

**Exemple 24 :1,2-Bis-(4-*tert*-butyl-phényl)-2-hydroxy-éthanone (WP101)**

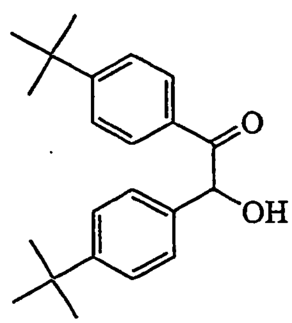

$C_{22}H_{28}O_2$ M = 324.462 $g.mol^{-1}$

A une solution de 4-*tert*-butylbenzaldéhyde (115 g, 708.9 mmol) dans un mélange méthanol (300 mL) / eau (40 mL) est ajoutée lentement une solution de cyanure de potassium (4.61 g, 70.89 mmol) dans l'eau (14 mL). Le mélange est agité à 90 °C pendant 40 heures. Après refroidissement, le méthanol est concentré sous pression réduite et le résidu est repris dans du dichlorométhane et de l'eau. Après trois extractions au dichlorométhane, les phases organiques rassemblées sont séchées sur sulfate de magnésium, filtrées et concentrées sous pression réduite. Du pentane (environ 800 mL) est alors ajouté, et le précipité résultant est filtré, lavé plusieurs fois au pentane et séché pour donner 55.23 g (49%, non optimisé) de produit pur sous la forme d'un solide blanc. Analyses identiques à la littérature.

**Exemple 25 : 1,2-Bis-(4-*tert*-butyl-phényl)-éthane-1,2-dione (WP103)**

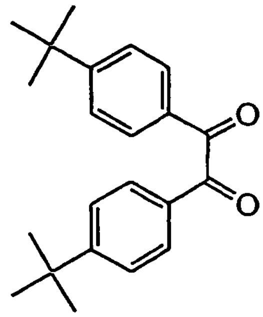

$C_{22}H_{26}O_2$ M = 322.4462 g.mol$^{-1}$

Une solution refroidie à 0 °C de WP101 préparé selon l'exemple 24 (12 g, 37.037 mmol) dans du dichlorométhane sec (350 mL) est ajoutée en 10 minutes une solution 15% en poids de périodinane de Dess-Markin dans le dichlorométhane (100 mL, environ 46.29 mmol). Le mélange est agité une nuit, puis dilué par du dichlorométhane et traité par une solution saturée d'hydrogénocarbonate de sodium. Après 10 minutes d'agitation, la phase organique est séparée et la phase aqueuse est extraite au dichlorométhane. Les phases organiques rassemblées sont lavées par une solution saturée de chlorure de sodium, filtrées et concentrées sous pression réduite. Le résidu obtenu est purifié par flash chromatographie sur gel de silice (pentane/dichlorométhane 4/1 à 1.5/1) pour donner 10.78 g (90%) d'une huile jaune qui se solidifie sous haut vide. Analyses identiques à la littérature.

**Exemple 26: Synthèse du 14-Bis[5,6-(4.4'-4-di-*tert* butyl)-diphényl-1,2,4-triazin-3-yl] benzène (WP104)**

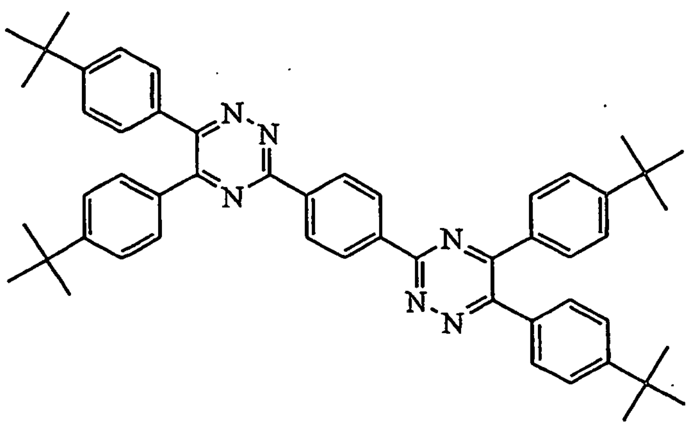

$C_{52}H_{56}N_6$ M = 765.0546 g.mol$^{-1}$

A une suspension de WP103 préparé selon l'exemple 25 (10.78 g, 33.47 mmol) dans l'éthanol (250 mL) est ajoutée la téréphthalamidrazone WP 29 préparée selon l'exemple 3 (2.924 g, 15.217 mmol) en une fois. Le milieu est chauffé à reflux pendant 15 heures. Après retour à température ambiante, le précipité obtenu est filtré, lavé à l'éthanol et séché pour donner 10.56 g (93%) d'un solide jaune. $\delta_H$ (300 MHz, $CDCl_3$) 1,35 (s, 36H), 7,42 (m; 8H), 7,63 (d; *J8,1;* 4H), 7,71 (d; *J* 8,4; 4H), 8,85 (s; 4H); MS (Electrospray) *m/z* 1789 (3M+H$^+$, 65%), 1530 (2M+H$^+$, 71%), 765 (MH$^+$, 100%).

**Exemple 27 : 1,2-Bis-(4-dodécylphényl)-éthane-1,2-dione[(7)] (WP105)**

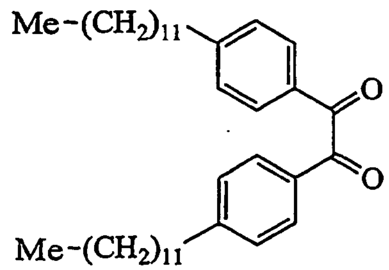

$C_{38}H_{58}O_2$ M = 546.8750 g.mol$^{-1}$

A une solution de 30 mL de THF sec et de *s*-BuLi (1.3 M in cyclohexane, 47.7 mL, 62.065 mmol) refroidie à -78 °C et sous atmosphère d'azote est ajoutée lentement une solution de 4-bromo-dodécylbenzène WP 60 préparée selon l'exemple 16 (20.19 g, 62.065 mmol) dans le THF (80 mL). Après une heure d'agitation, le mélange est transféré à l'aide d'une canule à une suspension de 1,4-diméthylpipérazine-2,3-dione (107 mg, 0.7539 mmol) dans le THF (2.7 mL) refroidi à -40 °C. Le milieu est agité pendant 15 heures puis traité par HCl 2N. Après dilution au dichlorométhane et agitation, la phase organique est séparée, lavée par HCl 2N puis par de l'eau, et séchée sur sulfate de magnésium. Après filtration et concentration, le résidu est purifié par flash chromatographie sur gel de silice (pentane/acétate d'éthyle 100/0; 10/1; 4/1; 3/1; 2/1) pour donner 2.77 g (18%) d'une huile jaune. Analyses identiques à la littérature.

**Exemple 28: Synthèse du 1,4-Bis[5,6-bis(4-dodécylphényl)-1,2,4-triazine-3-yl]benzène (WP107)**

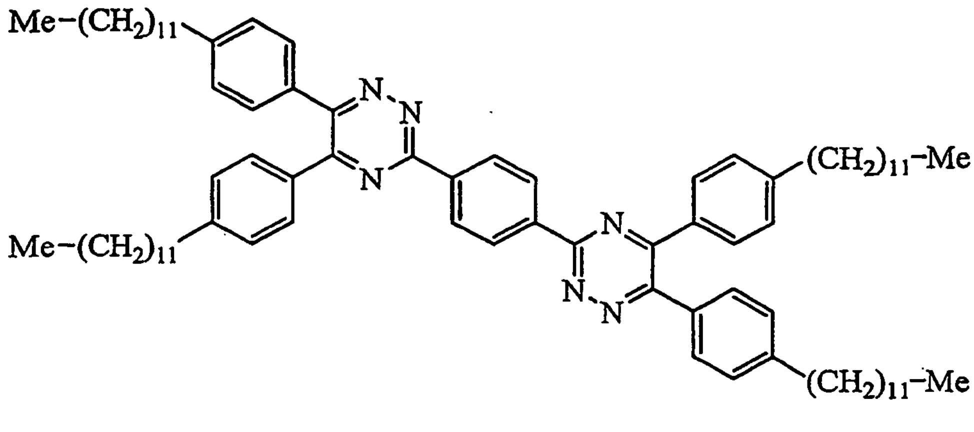

$C_{84}H_{120}N_6$ M = 1213.9122 g.mol$^{-1}$

A une solution de WP105 préparé selon l'exemple 27 (1.649 g, 3.147 mmol) dans l'éthanol (70 mL) est ajoutée la téréphthalamidrazone WP 29 préparée selon l'exemple 3 (275 mg, 1.431 mmol) en une fois. Le milieu est chauffé à reflux pendant 15 heures. Après retour à température ambiante, le précipité obtenu est filtré, lavé à l'éthanol et séché pour donner 1.38 g (80%) d'un solide jaune. $\delta_H$ (300 MHz, $CDCl_3$) 0,87 (m; 12H), 1,20-1,40 (m; 72H), 1,50-1.70 (m, 8H), 2,63 (t; *J* 7,5; 8H), 7,20 (m; 8H), 7,57 (d; *J 8,1;* 4H), 7,65 (d; *J 8, 4;* 4H); MS (Electrospray) *m/z* 1213.9 ($MH^+$, 100%).

**Exemple 29 : Synthèse du Biphényl-4,4'-dicarboximidate de diéthyle (WP129)**

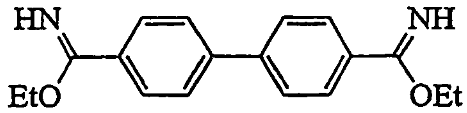

$C_{18}H_{20}N_2O_2$ M = 296,3682 g.mol$^{-1}$

A une suspension de téréphthalonitrile (2,5g; 12,24 mmol) dans de l'éthanol absolu (30 ml) refroidie à 0 °C est effectué un bullage de HCl gazeux pendant 24 heures, durant lesquelles la température est remontée à l'ambiante. Le solide blanc obtenu est alors filtré et lavé à l'éthanol. La neutralisation de ce sel dissout dans un minimum d'eau à 0 °C est effectuée en ajoutant à 0 °C une solution de $K_2CO_3$ (30% en poids) jusqu'à pH basique. Le solide blanc obtenu est dissout dans du dichlorométhane. La phase aqueuse est séparée puis la phase organique est séchée sur $MgSO_4$, filtrée et concentrée pour donner un solide blanc (rendement > 90%). $\delta_H$ (300 MHz, $CDCl_3$) 1,44 (t; *J* 7,2; 6H), 4.34 (q; *J* 7,2; 4H), 7.63 (d, *J 8,7;* 4H), 7.83 (d, *J 8, 7;* 4H).

**Exemple 30 : 4-(1-oxo-4-méthyl-pentyl)bromobenzène[(9)] (WP132)**

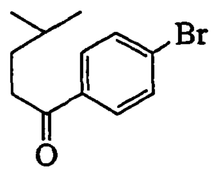

$C_{12}H_{15}BrO$ M = 255.1539 g.mol$^{-1}$

Un mélange d'acide 4-méthyl valérique (15 g, 129.1 mmol) et de chlorure de thionyle (10.8 mL, 148 mmol) est chauffé à reflux pendant 90 minutes. L'excès de chlorure de thionyle est distillé sous pression réduite, puis le résidu est repris dans 48 mL de bromobenzène. Après refroidissement. à 0 °C, du chlorure d'aluminium anhydre (13.8 g, 103.5 mmol) est ajouté à la solution. Le mélange est agité à température ambiante pendant 80 heures, puis traité par addition d'eau glacée, puis par 20 mL d'HCl concentré. La phase organique est séparée et la phase aqueuse extraite par $Et_2O$. Les phases organiques rassemblées sont lavées successivement par de l'eau puis par une solution saturée de chlorure de sodium, séchées sur $MgSO_4$, filtrées et concentrées sous pression réduite. Le résidu est purifié a) soit par distillation (environ 80 °C/0.01 mm), b) soit par chromatographie sur gel de silice (pentane 100% puis pentane/acétate d'éthyle 10/1) suivie d'une filtration, lavage à l'eau et séchage, pour donner 20.337 g (62%, non optimisé) d'un solide incolore. Analyses identiques à la littérature.

**Exemple 31 : 4-(1,1,4-Triméthyl-pentyl)bromobenzène[(10)] (WP133)**

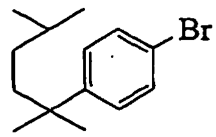

$C_{14}H_{21}Br$ $M = 269.2239\ g.mol^{-1}$

A une solution de cétone WP132 préparée selon l'exemple 30 (2.675 g, 10.49 mmol) dans du chlorobenzène (4 mL) et de l'eau (100 μL) sous atmosphère d'azote et refroidie à 0 °C est ajoutée lentement une solution 2M de triméthylaluminium dans l'hexane (10,5 mL, 2 éq.). L'hexane est ensuite distillé puis la solution est chauffée à reflux pendant 80 heures. Après refroidissement, le mélange est traité par lente addition d'eau, puis par HCl 2N et chauffé jusqu'à dissolution des sels. Après refroidissement, le mélange est extrait plusieurs fois par $Et_2O$. Les phases organiques rassemblées sont successivement lavées par de l'eau puis par une solution saturée de NaCl, séchées sur sulfate de magnésium, filtrées et concentrées sous pression réduite. Le résidu est alors distillé (environ 100 C/0.16 mm) pour donner un mélange de produits qui est dissout dans 10 mL de dichlorométhane, traité par 1.0 g de *m*-CPBA et agité pendant 12 heures. Après concentration sous pression réduite, le résidu est purifié par chromatographie sur gel de silice (pentane 100%) pour donner 1.143 g (40%, non optimisé) d'un liquide incolore. Analyses identiques à la littérature.

**Exemple 32 : Synthèse de la Bis-amidrazone (WP134)**

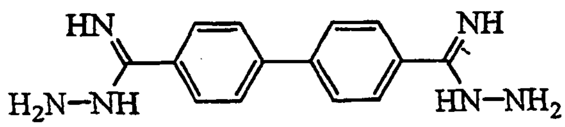

$C_{14}H_{16}N_6$ $M = 268,3206\ g.mol^{-1}$

A une suspension de WP129 préparé selon l'exemple 29 (1,296 g; 4,37 mmol) dans de l'éthanol absolu (7 mL) est ajoutée de l'hydrazine monohydratée (637 μL; 13,1 mmol) durant 10 minutes. Après 24 heures d'agitation, le précipité est filtré, lavé successivement à l'éthanol puis à l'éther diéthylique et séché pour donner 1,077g (92%) d'un solide jaune. $\delta_H$ (300 MHz, DMSO) 5,00 (brs; 4H), 5,62 (s, 4H), 7,66 (d; *J* 8,7; 4H), 7,78 (d; *J* 8, 7; 4H).MS (Electrospray) *mlz* 269 ($MH^+$, 100%).

**Exemple 33: Synthèse de la 1,4-bis-[5,6-(4,4'-diphényl-1,2,4-triazine-3-yl]diphényle (WP135)**

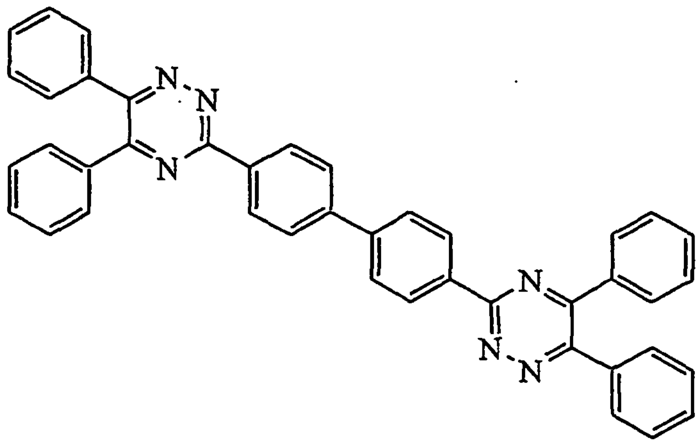

$C_{42}H_{28}N_6$ $M = 616,7234\ g.mol^{-1}$

A une solution de benzil (589 mg, 2,79 mmol) dans l'éthanol (20 mL) est ajouté WP134 préparé selon l'exemple 32 (300 mg, 1,12 mmol) en une fois. Le milieu est chauffé à reflux pendant 15 heures. Après retour à température ambiante, le précipité obtenu est filtré, lavé successivement à l'éthanol puis à l'éther diéthylique et séché pour donner 548 mg (80%) d'un solide jaune. $\delta_H$ (300 MHz, DMSO-*d*, 120°C) 7,40-7,57 (m; 12H), 7,60 (m; 4H), 7,68 (m; 4H), 8,10 (d; 4H), 8,77 (d; 4H). MS (Electrospray) *m/z* 617 ($MH^+$, 10%), 457 (26%), 190 (100%).

**Exemple 34: Synthèse de la 1,2-Bis-[4-(2-ethyl-hexyloxy)-phenyl]-ethane-1,2-dione (WP141)**

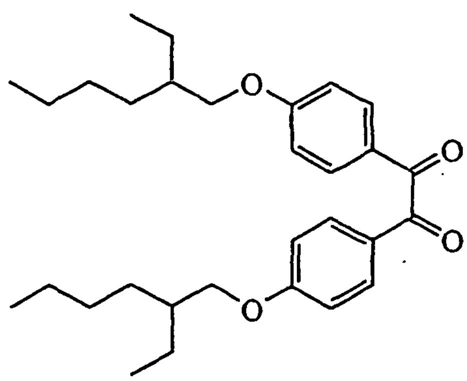

$C_{30}H_{42}O_4$ $M = 466,6594\ g.mol^{-1}$

A une solution de WP32 préparé selon l'exemple 5 (3,00 g; 12,4 mmol) dans le DMF (75 mL) est ajoutée de la soude (1,24 g; 30,8 mmol). Le mélange est agité 5 minutes à température ambiante, puis du bromure de 2-éthylhexyle (6,6 mL; 37,1 mmol) est ajouté en 1 minute. Après 60 heures d'agitation à 60 °C, le milieu est refroidi. Le mélange est jeté sur de l'eau glacée et extrait par de l'acétate d'éthyle. La phase organique est lavée plusieurs fois par une solution saturée de bicarbonate de sodium, séchée sur sulfate de sodium, filtrée et concentrée. Le résidu est purifié par chromatographie-flash sur gel de silice (pentane100% puis pentane/acétate d'éthyle 23/1) pour donner 4,879g (85%) d'un liquide jaune. $\delta_H$ (300 MHz, $CDCl_3$) 0,90 (m; 12H), 1,25-1,35 (m; 8H), 1,45 (m, 8H), 1,75 (m, 2H), 3,91 (d; *J 6,0;* 4H), 6,94 (d; *J 9,0;* 4H), 7,92 (d; *J 9,0;* 4H); ; MS (Electrospray) *m/z* 954 (2M+$H^+$, 100%), 467 ($MH^+$, 33%).

**Exemple 35: Synthèse du 1,4-bis-[5,6-bis(4,2-éthylhexyloxyphényl)-1,2,4-triazine-3-yl]benzène (WP144)**

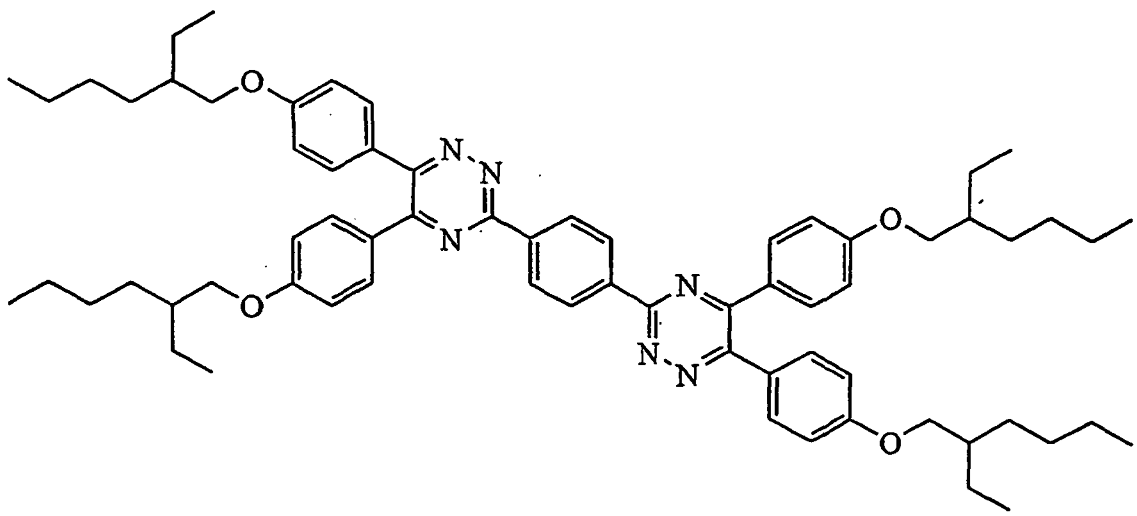

$C_{68}H_{88}N_6O_4$ . M = 1053,481 g.mol$^{-1}$

A une solution de WP141 préparé selon l'exemple 34 (1,821 g, 1,52 mmol) dans l'éthanol (40 mL) est ajoutée la téréphthalamidrazone WP 29 préparée selon l'exemple 3 (300 mg, 1,56 mmol) en une fois. Le milieu est chauffé à reflux pendant 15 heures. Après retour à température ambiante, le précipité obtenu est filtré, lavé successivement à l'éthanol puis à l'éther diéthylique et séché pour donner 1,452 g (88%) d'un solide jaune. $\delta_H$ (300 MHz, $CDCl_3$) 0,90 (m; 24H), 1,25-1,60 (m; 32H), 1,74 (m, 4H), 3,89 (d; *J 6, 0;* 8H), 6,92 (m, 8H), 7,63 (d; *J* 8,4; 4H), 7,74 (d; *J 8,4;* 4H), 8,82 (s, 4H). MS (Electrospray) *m/z* 1053 ($MH^+$, 100%).

**Exemple 36 : Synthèse de la 1,2-Bis-[4-(1-méthyl-heptyloxy)-phényl]-éthane-1,2-dione (WP145)**

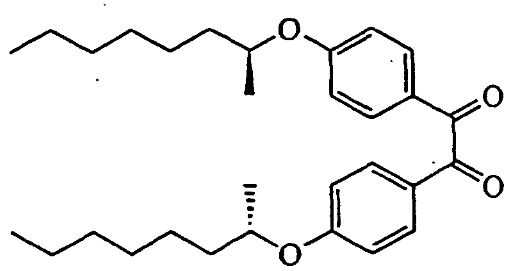

$C_{30}H_{42}O_4$ M = 466,6594 g.mol$^{-1}$

A une solution de WP32 préparé selon l'exemple. 5 (286 mg; 1,184 mmol) dans le DMF (6 mL) est ajouté du carbonate de potassium (900 mg; 7,1 mmol), puis du tosylate de (S)-2-octyle(10) (740 mg, 2,6 mmol). Après 15 heures d'agitation à 50 ˚C, le milieu est refroidi, puis jeté sur de l'eau glacée et extrait par de l'acétate d'éthyle. La phase organique est lavée plusieurs fois par une solution saturée de bicarbonate de sodium, séchée sur sulfate de sodium, filtrée et concentrée. Le résidu est purifié par chromatographie-flash sur gel de silice (pentane100% puis pentane/acétate d'éthyle 20/1) pour donner 406 mg (74%) d'une huile jaune. $\delta_H$ (300 MHz, $CDCl_3$) 0,80-0,93 (m; 6H), 1,20-1,40 (m; 12H), 1,31 (d; *J*. 6; 6H), 1,55 (m, 4H), 1,73 (m, 4H), 4,46 (m; 2H), 6,91 (d; *J 9,3*; 4H), 7,92 (d; *J 9,3*; 4H); ; MS (Electrospray)

*m/z* 955 (2M+$Na^+$, 100%), 467 ($MH^+$, 56%).

**Exemple 37: Synthèse du 1,4-bis[5,6-bis(4,1-méthyheotyloxyphényl)-1,2,4-triazine-3-yl]benzène(WP149)**

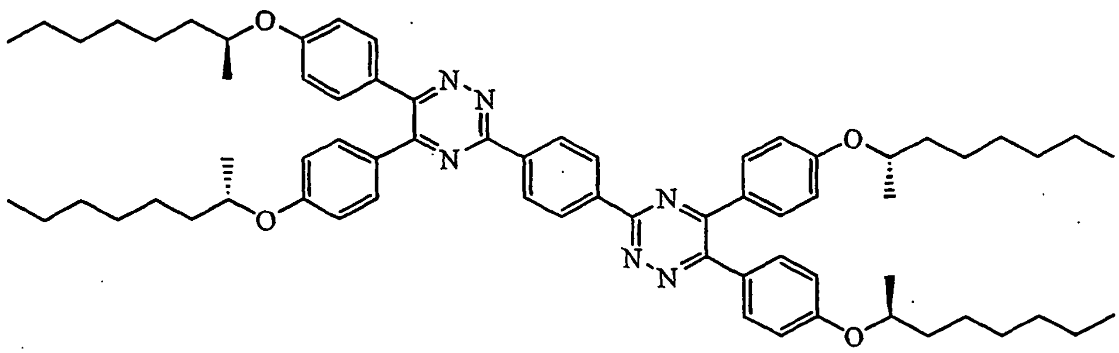

$C_{68}H_{88}O_4N_6$ M = 1053,4 $g.mol^{-1}$

A une solution de WP145 préparé selon l'exemple 36 (398 mg, 0,85 mmol) dans l'éthanol (8 mL) est ajoutée la téréphthalamidrazone WP 29 préparée selon l'exemple 3 (69 mg, 0,355 mmol) en une fois. Le milieu est chauffé à reflux pendant 15 heures. Après retour à température ambiante, le précipité obtenu est filtré, lavé successivement à l'éthanol puis au pentane froid et séché pour donner un solide jaune (rendement non calculé). $\delta_H$ (300 MHz, $CDCl_3$) 0,80-0,95 (m; 12H), 1,20-1,53 (m; 24H), 1,31 (d; *J.* 6; 12H), 1,60 (m, 8H), 1,75 (m, 8H), 4,42 (m; 4H), 6,88 (d; *J 9,0;* 4H), 6,91 (d; *J 9,0;* 4H), 7,63 (d; *J 9,0;* 4H), 7,73 (d; *J 9,0;* 4H), 8,82 (s, 4H). MS (Electrospray) *m/z* 1053 ($MH^+$, 100%).

**Exemple 38 : Synthèse de la 1,2-Bis-[4-(1,1,4-triméthyl-pentyl)-phényl]-éthane-1,2-dione (WP150)**

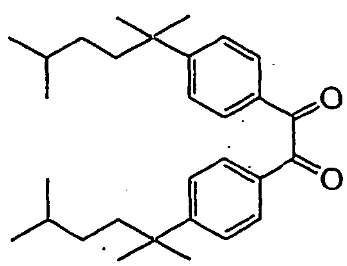

$C_{30}H_{42}O_2$ M = 434.6606 $g.mol^{-1}$

Une solution de s-BuLi (1.3 M in cyclohexane, 2.9 mL, 3.71 mmol) est ajoutée lentement à une solution de WP133 préparé selon l'exemple 31 (1.0 g, 3.71 mmol) dans du THF anhydre (4.6 mL) à -78 °C et sous atmosphère d'azote. Après une heure d'agitation au cours desquelles 3 mL supplémentaires de THF sont ajoutés, le mélange est réchauffé à environ 0°C puis transféré à l'aide d'une canule à une suspension de 1,4-diméthylpipérazine-2,3-dione (240 mg, 1.688 mmol) dans le THF (6 mL) refroidie à 0 °C. Après retour à température ambiante, le milieu est agité pendant 3 heures puis traité par du HCl 2N. Après dilution par $Et_2O$, la phase organique est séparée et la phase aqueuse extraite 2 fois par $Et_2O$. Les phases organiques rassemblées sont lavées par de l'eau puis par une solution saturée de NaCl et séchées sur sulfate de magnésium. Après filtration et concentration, le résidu est purifié par flash chromatographie sur gel de silice (pentane/acétate d'éthyle 100/0; 6/1; 4/1) pour donner 523 mg (71%, non optimisé) d'une huile jaune. $\delta_H$

(300 MHz, $CDCl_3$) 0,80 (d; *J 6, 0;* 12H),0,85-0,97 (m; 4H), 1,3 (s, 12H), 1,40 (sept.; *J 6, 0;* 2H), 1,55-1,70 (m, 4H), 7,45 (d; *J 8, 7;* 4H), 7,91 (d; *J 8, 7;* 4H).

**Exemple 39 : Synthèse du bis(4-(1,1,4-triméthylpentyl)phenyl)-1,2,4-triazine-3-yl]benzène (WP151)**

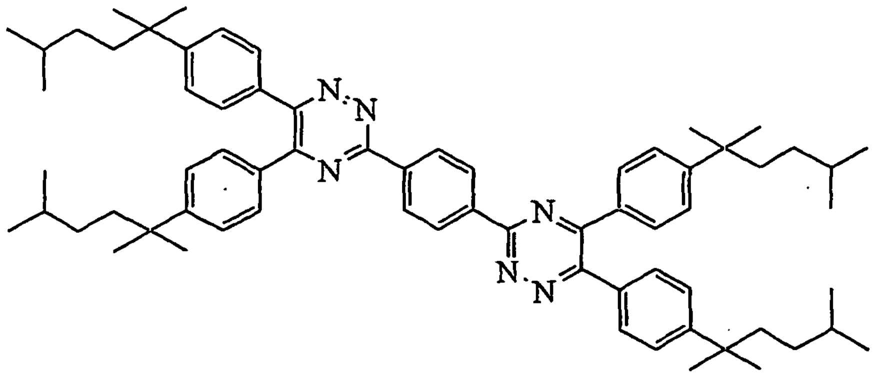

$C_{68}H_{88}N_6$ $M = 989.4834\ g.mol^{-1}$

A une solution de WP150 préparé selon l'exemple 38 (520 mg, 1.196 mmol) dans l'éthanol (10 mL) est ajoutée la téréphthalamidrazone (104 mg, 0.544 mmol) en une fois. Le milieu est chauffé à reflux pendant 15 heures. Après retour à température ambiante, le précipité obtenu est filtré, lavé à l'éthanol et séché pour donner 348 mg (65%, non optimisé) d'un solide jaune. $\delta_H$ (300 MHz, $CDCl_3$) 0,81 (d; *J 6,0;* 24H),0,90-0,10 (m; 8H), 1,31 (s, 24H), 1,40 (sept.; *J 6,0;* 4H), 1,53-1,70 (m, 8H), 7,33 (d; *J 8,7*; 4H), 7,34 (d; *J 8, 7; 4H),* 7,58 (d; *J 8,7*; 4H), 7,66 (d; *J* 8, 7; 4H). MS (Electrospray) *m/z* 1978 ($2M+H^+$,30%), 989 ($MH^+$, 100%).

**Références bibliographiques des synthèses :**

(1) G. Pitet, H. Cousse, G. Mouzin, Boll, Chim, Farm., 1980, 119, 469
(2) F. Lu, Y. Wang, L. Xing, Gaofenzi Tongxun 1981, 5, 319
(3) X. Yi, G. Wu, F. Lu A. Tang, J. Appl. Point. Sci. 2001, 907, 82
(4) H. Simbürger, W. Kern, K. Hummel, C. Hagg, Polym. 2000, 41, 7883
(5) L. Brunsvelt, H. Zhang, M. Glasbeek, J. A. J. M. Vekemans, E. W. Meijer, J. Am. Chem. Soc. 2000, 122, 6175
(6) T. Tagusari, Y. Honda, *Jpn. Kokai Tokkyo Koho* **1992,** JP 04279546
(7) M. Wehmeier, M. Wagner, K. Müllen, Chem. Eur. J., 2001, 7, 2197
(8) T. Shono, M. Masahiro, S. Matsumura, N. Asano, *Jpn. Tokkyo Koho* **1968,** JP 43015992
(9) US Patent 1993 n° 5202471
(10) M. S. Alnajjar, H. G. Kuivila, J. Am. Chem. Soc. 1985, 107, 422.


On trouvera ci-après les études physico-chimiques réalisées sur les composés objets de la présente invention, en comparaison avec les filtres commerciaux suivants :

PARSOL 1789®:

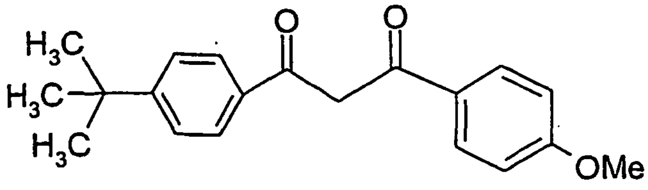

PARSOL MCX®:

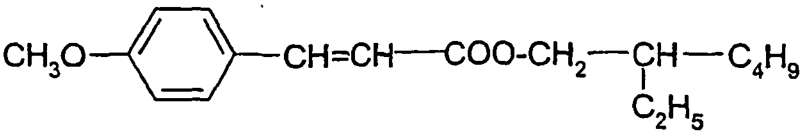

TINOSORB S®:

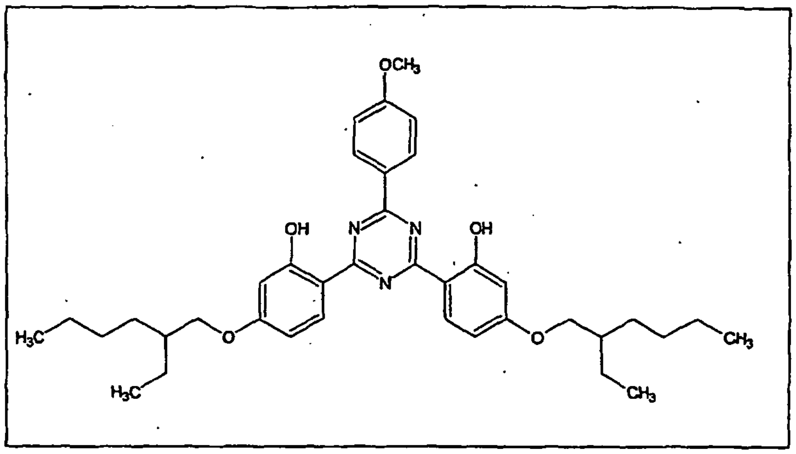

TINOSORB M®:

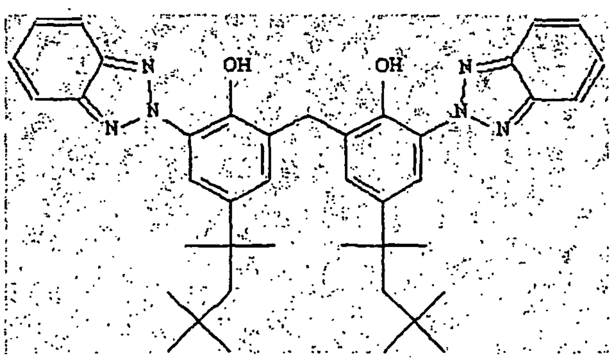

**Exemple 40 : Caractéristiques spectrales des produits : coefficient d'extinction molaire et l'absorbance spécifique**

Le calcul du coefficient d'extinction molaire (ε) se fait à partir de la loi de Beer-Lambert :

$$Log_{10}\left(\frac{I_0}{I}\right) = \varepsilon.l.c = A$$

Où:

A= absorbance
$I_0$=ntensité de la lumière incidente
I= intensité de la lumière transmise
ε= coefficient d'extinction molaire ( ou absorbance molaire ) en $M^{-1}$. $cm^{-1}$
1= longueur de la cuve en cm
c= concentration en mol. $L^{-1}$

Le coefficient d'extinction molaire peut-être exprimé par rapport à une masse donnée de produit. Il permet alors de pouvoir comparer les coefficients d'extinction entre eux pour une même quantité de produit donné. Cette quantité est de 1% en poids. Le coefficient d'extinction molaire devient alors l'*absorbance spécifique* ( $A_{1cm}^{1\%}$ ).

Il s'exprime comme il suit :

$$A_{1cm}^{1\%} = \varepsilon.\frac{10}{M}$$

Où:

$A_{1cm}^{1\%}$ : l'absorbance spécifique

ε=coefficient d'extinction molaire
M= masse molaire

Les caractéristiques spectrales des composés en comparaison avec des filtres commerciaux à une concentration de 10 μg.$ml^{-1}$ sont regroupées dans les tableaux 2-1 et 2-2.

**Mode opératoire :** Les produits sont dissous dans de l'acétate d'éthyle à une concentration de 10 J.Lg.$ml^{-1}$. Les spectres sont effectués à l'aide d'un spectrophotomètre (Varian CARY 50 Scan) à double faisceau entre 290 nm et 400 nm.

**TABLEAU 2-1**

| | Coefficient d'extinction molaire maximal | | | | Absorbance spécifique maximale | | | |
|---|---|---|---|---|---|---|---|---|
| **Molécules** | **UVC** | **UVB** | **UVA** | **Visible** | **UVC** | **UVB** | **UVA** | **Visible** |
| Parsol 1789® | 8633 à 275 | | 34100 à 360 | | 278 à 275 | | 1100 à 360 | |
| Parsol MCX® | | 24600 à 310 | | | | 848 à 310 | | |
| Tinosorb S® | | 47150 à 310 | 49580 à345 | | | 751 à 310 | 789 à 345 | |
| Tinosorb M® | | 36600 à 305 | 36400 à 345 | | | 556 à 305 | 552 à 345 | |

**TABLEAU 2-2**

| Composés de formule (I) | | | | | | | | |
|---|---|---|---|---|---|---|---|---|
| | Coefficient d'extinction molaire maximal | | | | Absorbance spécifique maximale | | | |
| Molécules | UVC | UVB | UVA | Visible | UVC | UVB | UVA | Visible |
| WP30 | | | 50200 à 325 | | | | 928 à 325 | |
| WP35 | | 29600 à 310 | 27800 à 355 | | | 490 à 310 | 460 à 355 | |
| WP39 | | | 48200 à 335 | | | | 808 à 335 | |
| MT41 | | 72000 à 305 | 91400 à330 | | | 527 à 305 | 669 à 330 | |
| WP52 | | | 57300 à 330 | | | | 669 à 330 | |
| WP76 | | 54000 à 290 | | | | 998 à 290 | | |
| WP89 | | | 47700 à 330 | | | | 778 à 330 | |
| WP96 | | | 56000 à 335 | | | | 638 à 335 | |
| WP100 | | | 43400 à 300 | | | | 640 à 100 | |
| WP104 | | 90000 à 300 | | | | 1176 à 300 | | |
| WP107 | | 92000 à 300 | 95000 à 335 | | | 757 à 335 | 783 à 300 | |
| WP135 | | | 57000 à 330 | | | | 924 à 330 | |
| WP144 | | 66000 à 315 | 64000 à 355 | | | 626 à 315 | 607 à 355 | |
| WP149 | | 56000 à 315 | 59000 à355 | | | 532 à 315 | 560 à 355 | |
| WP151 | | | 60500 à 340 | | | | 611 à 340 | |

**Exemple 41 :**

Les produits testés sont classés suivant la répartition spectrale dans l'UVA et l'UVB dans un domaine allant de 290 à 400nm.

Il est possible de les différencier en fonction de leur répartition spectrale : Produits à spectre étroit :

- Les produits absorbant dans la zone comprise entre 280 nm et 320 nm (UVB)
- Les produits absorbant dans la zone comprise entre 320 nm et 400 nm (UVA)

Produits à large spectre :

- Les produits absorbant dans l'UVB (280 nm - 320 nm) et l'UVA-II (380 nm - 360 nm)
- Les produits couvrant l'UVB et l'UVA ( 280 nm - 400 nm).

**Exemple 41-1: Répartition spectrale des composés de formule (I)**

WP89, WP96, WP100, WP104, WP107, WP135, WP144, WP149, WP151, WP35; WP39, WP41, WP52 et WP30 absorbent dans l'UVB et l'UVA (voir figures 1 et 2).
WP76 absorbe dans l'UVB (voir figure 3).

**Exemple 41-2 :**

Le tableau 3 résume les répartitions spectrales des composés testés.

**TABLEAU 3**

| Molécules | UVB 280-320 nm | UVA-II 320-340 nm | UVA-I 340-400 nm | Pics d'absorption ( nm) |
|---|---|---|---|---|
| Parsol 1789® | Absorption nulle | [illegible] | Très forte absorption | 360 |
| WP76 | Très forte absorption | Forte absorption | Absorption nulle | 290 |
| Parsol® | Très forte absorption | Absorption nulle | Absorption nulle | 310 |
| Tinosorb S® | Très forte absorption | Très forte absorption | Très forte absorption | 310 et 345 |
| Tinosorb M® | Très forte absorption | Très forte absorption | Très forte absorption | 305 et 345 |
| WP30 | Très forte absorption | Très forte absorption | Très forte absorption | 325 |
| WP35 | Très forte absorption | Très forte absorption | Absorption moyenne | 355 |
| WP39 | Très forte absorption | Très forte absorption | Absorption moyenne | 335 |
| WP41 | Très forte absorption | Très forte absorption | Très forte absorption | 205 et 330 |
| WP52 | Très forte absorption | Très forte absorption | Absorption faible | 330 |
| WP89 | Très forte absorption | Très forte absorption | Absorption moyenne | 330 |
| WP96 | Très forte absorption | Très forte absorption | Absorption moyenne | 335 |
| WP100 | Très forte absorption | Forte absorption | Absorption faible | 300 |
| WP104 | Très forte absorption | Forte absorption | Absorption moyenne | 300 |
| WP107 | Très forte absorption | Très forte absorption | Absorption moyenne | 300 et 335 |
| WP135 | Très forte absorption | Très forte absorption | Absorption moyenne | 330 |
| WP144 | Très forte absorption | Très forte absorption | Très forte absorption | 315 et 355 |
| WP149 | Très forte absorption | Très forte absorption | Très forte absorption | 315 et 355 |
| WP151 | Très forte absorption | Très forte absorption | Absorption moyenne | 340 |

| Motif | Légende |
|---|---|
| (pointillé) | Très forte absorption |
| (lignes horizontales) | Forte absorption |
| (trame dense) | Absorption moyenne |
| (lignes verticales) | Absorption faible |
| (blanc) | Absorption nulle |

**Exemple 42 : Evaluation du facteur de protection solaire (FPS) *in vitro* dans unsolvant chimique**

Les méthodes *in vitro* de détermination de l'efficacité protectrice des produits solaires consistent à mesurer par spectrophotométrie de transmission le spectre d'absorption du filtre en solution ou du produit appliqué sur un substrat visant à simuler le relief de la peau. L'efficacité contre les rayons UVB, UVA ou les deux, ou leurs effets sur la réponse cutanée, sont ensuite déterminés par le calcul, prenant en compte ou non le spectre d'action des radiations UV pour le dommage considéré.

La méthode de Sayrel Agin et Diffey/ Robson, pratiquée depuis les années 1990, préconise une mesure comparative, à l'aide d'un spectroradiomètre à sphère intégratrice, de la transition de 290 nm à 400 nm par bandes de 5 nm, l'échantillon étant soumis au rayonnement UV d'une source stable et connue couvrant la totalité du spectre UV ( xénon non filtré).

Diffey et Robson pour évaluer par le calcul est la réponse érythèmale. La formule est la suivante :

$$FPS = \frac{\left(\sum_{290}^{400} E(\lambda) * \varepsilon\right)}{\left(\sum_{290}^{400} \frac{E(\lambda) * \varepsilon}{\overline{FPM}(\lambda)}\right)}$$

E(λ)= Irradiation spectrale en $W.m^{-2}.nm^{-1}$ à 40°N soleil au zénith angle 20° ε= Capacité érythémateuse

$$\overline{FPM}(\lambda) = \frac{\left(\sum_{i=1}^{N} FPM(\lambda)i\right)}{N(\lambda)}$$

N(λ)= nombre de valeurs pour une longueur d'onde déterminée

La formule de Diffey et Robson a permis de déterminer le FPS à partir de la mesure de la transmittance entre 290 nm et 400 nm. La transmittance est mesurée en solution dans de l'acétate d'éthyle à une concentration de 10μg.ml$^{-1}$ à l'aide d'un spectrophotomètre UV-visible (Varian CARY 50 Scan).

$$FPM(\lambda) = \frac{1}{T(\lambda)}$$

T(λ)= la transmitance à une longueur d'onde λ.

Les résultats des mesures effectuées sont rassemblés dans le tableau 5.

| TABLEAU 4 | |
|---|---|
| **Mesure du FPS in vitro dans un solvant chimique** | |
| **Molécules** | **FPS** |
| **Parsol MCX®** | 20,78 |
| **Parsol 1789®** | 51,1 |
| **Tinosorb M®** | 54,07 |
| **Tinosorb S®** | 76,19 |
| **Composés de formule (I)** | |
| **WP96** | 68,02 |
| **WP104** | 79,35 |
| **WP149** | 74,1 |
| **WP151** | 67,2 |

**Exemple 43 : Etude de la photostabilité en solution dans un solvant chimique**

Nous avons utilisé un Suntest CPS+ (ATLAS, Linsengenicht/Altenhasslan, Germany). Le Suntest permet de reproduire le spectre solaire et donc de réaliser-des expositions en section intérieure en temps voulu et sans contraintes météorologiques.

Réglage de la DEM (Dose Erythémale Minimale) :

L'irradiance du simulateur solaire a été soigneusement mesurée avec un spectroradiomètre (MSS 2044, Bielefeld, Germany). Les intensités des UVB et des UVA étaient respectivement de 0.49 mW/cm$^2$ et 6.32 mW/cm$^2$. La valeur de la DEM définie par le COLIPA est de 5,6 J/cm$^2$ en UV totaux (22). Les UV totaux (UVA+UVB) représentent 14,8% de l'énergie délivrée par la lampe (puissance 460W/m$^2$). Une dose d'irradiation équivalente à 1 DEM correspond à 37,83 J/cm$^2$ (en spectre total) délivrés par la lampe.

La durée d'essai avec le Suntest se calcule grâce à la formule suivante :

$$t = H / E$$

avec

E : Eclairement énergétique en $W/m^2$
H : Dose d'irradiation en $J/m^2$
t : Durée de l'essai en s.

Le réglage de la DEM sur le Suntest et la correspondance avec l'ensoleillement de 3 stations balnéaires sont indiqués au Tableau 6.

**TABLEAU 6**

| Ensoleillement | Extrême | Intense | Moyen |
|---|---|---|---|
| Lieu (21 juin) | Agadir | Toulon | La Baule |
| Nombre DEM/j | 20 | 10 | 5 |
| Dose d'irradiation correspondante sur le Suntest (en $J/m^2$) | 7566000 | 3783000 | 1891500 |
| Durée de l'essai | 4H34 | 2H17 | 1H08 |

**Mode opératoire :**

Les solutions de composés sont préparées en une concentration de 500 µg/mL dans le méthanol. 50 µL (soit 25 µg) de chacune des solutions sont déposés dans des cristallisoirs, puis irradiés dans le Suntest à 5, 10 et/ou 20DEM. Un témoin non irradié est préparé (dépôt de 50 µL de solution et ajout de 2,450 mL de méthanol). Le solvant s'évaporant lors de l'irradiation, les produits sont repris dans 2,5 ml de méthanol. Après l'irradiation, l'absorbance de chaque solution est mesurée au spectrophotomètre UV-visible (Varian CARY 50 Scan).

Les résultats de mesure de la photostabilité des composés de formule (I) sont regroupés dans le tableau 7.

**TABLEAU 7**

| | **Photostabilité à 5 DEM** | | | **Photostabilité à 10 DEM** | | |
|---|---|---|---|---|---|---|
| **molécule** | **moyenne** | **écart-type** | **écart-type relatif** | **moyenne** | **écart-type** | **écart-type relatif** |
| VPP30 | **77,75** | 0,78 | 1,00% | **56,12** | 0,54 | 137,83% |
| WP76 | **69,23** | 1,31 | 1,89% | **62,53** | 0,91 | 137,39% |
| WP96 | **83,30** | 5,94 | 7,13% | **74,35** | 4,15 | 138,07% |
| WP104 | **93,70** | 4,24 | 4,53% | **88,55** | 2,97 | 138,43% |
| WP107 | **89,27** | 1,46 | 1,64% | **84,75** | 1,02 | 138,29% |
| WP135 | **57,45** | 1,63 | 2,83% | **33,66** | 1,13 | 136,58% |
| WP149 | **80,40** | 1,56 | 1,93% | **38,16** | 1,09 | 137,95% |
| WP151 | **76,16** | 4,30 | 5,64% | **70,15** | 3,00 | 137,76% |

**Exemple 44 : Etude de solubilité**

Les résultats de solubilité dans des solvants ou excipients utilisés en cosmétique sont regroupés dans le tableau 8.

**TABLEAU 8**

| **Composés de formule (I)** | | |
|---|---|---|
| **Molécules** | **Excipient** | **Solubilité (%)** |
| WP96 | **Myritol 318®**<br>**Finsol** V **NT®**<br>**PEG400®** | 0,1<br>1<br>2 |
| WP107 | **Finsol V NT® 1**<br>**PEG400®** | 1<br>1 |

(suite)

| Composés de formule (I) | | |
|---|---|---|
| **Molécules** | **Excipient** | **Solubilité (%)** |
| | **Arlasolve DMI®** | 1 |
| WP149 | **Myritol 318®**<br>**Finsol VNT®**<br>**Adipate d'isopropyle** | 2,5<br>4,5<br>2 |
| WP151 | **Myritol 331®**<br>**Finsol V NT**<br>**Adipate d'isopropyle**<br>**PEG400®** | 2<br>5<br>1<br>0,5 |
| **Arlasolve DNI®** = Diméthyl Isosorbide<br><br>**Finsol V NT®=** C12-C15 alkyl benzoate<br><br>**Myritol 318®=** Caprilic/ Capric Triglycérides<br><br>**Myritol 331®=** Cocoglycérides (Glycéryl esters et dérivés)<br><br>**PEG400®** = Polyéthylèneglycol (n=400) | | |

**TABLEAU 9**

| Composés de formule (I) | | |
|---|---|---|
| **Molécules** | **Solubilités** | |
| | **Soluble** | **Insoluble** |
| WP30 | DMSO ou DMF chaud Acétate d'éthyle (PS) | Eau<br>MeOH<br>$CHCl_3$<br>Acétone<br>Acétonitrile<br>Benzène<br>Myritol<br>Transcutol |
| WP35 | DMSO 3,5 %<br>Transcutol 0.5 % | MeOH<br>$CHCl_3$<br>Acétate d'éthyle<br>Hexane<br>Myritol |
| WP38 | Benzène<br>Toluène<br>Myritol<br>Transcutol | MeOH<br>DMC<br>AcOEt |
| WP39 | DMSO ou DMF chaud | Eau<br>MeOH<br>$CHCl_3$<br>Acétone<br>Acétonitrile |

(suite)

| Composés de formule (I) | | |
|---|---|---|
| Molécules | Solubilités | |
| | Soluble | Insoluble |
| | | Benzène |
| WP40 | DMSO ou DMF chaud | Eau<br>MeOH<br>$CHCl_3$<br>Acétone<br>Acétonitrile<br>Benzène |
| WP41 | DMC<br>AcOEt (PS)<br>MeOH (PS)<br>DMSO 2,7%<br>Transcutol 0,1 % | EtOH<br>Hexane<br>Myritol |
| WP52 | DMSO ou DMF chaud | Eau<br>MeOH<br>$CHCl_3$<br>Acétone<br>Acétonitrile<br>Benzène<br>Myritol<br>Transcutol |
| WP76 | DMSO ou DMF chaud | Eau<br>MeOH<br>$CHCl_3$<br>Acétone<br>Acétonitrile<br>Benzène |
| WP89 | Acétate d'éthyle | |
| WP96 | DMC<br>$CHCl_3$<br>$Et_2O$<br>Toluène<br>Octanol<br>Transcutol 0,5 %<br>Méthanol | EtOH<br>Myritol<br>Acide acétique |
| WP100 | DMSO à chauds | DCM<br>$Et_2O$<br>Toluène<br>Octanol<br>EtOH<br>Myritol<br>Transcutol |
| WP104 | DCM<br>$CHCl_3$<br>Toluène<br>DMSO | EtOH<br>MeOH<br>1-Octanol<br>Acide acétique |

(suite)

| Composés de formule (I) | | |
|---|---|---|
| **Molécules** | **Solubilités** | |
| | **Soluble** | **Insoluble** |
| | Acétate d'éthyle | Myritol<br>Transcutol |
| WP107 | DCM<br>$CHCl_3$<br>Toluène<br>Octanol<br>Acétate d'éthyle<br>DMSO | EtOH<br>Acide acétique<br>MeOH<br>Myritol<br>Transcutol |
| DMSO : diméthylsulfoxyde<br>DMF : diméthylformamide<br>DCM : dichlorométhane | | |

**Exemple 45 : Exemple de formulation**

| Composition (émulsion H/E) | Quantité (g) |
|---|---|
| Sulfate de magnésium hydraté | 0,7 |
| Para méthoxy cinnamate d'éthyl hexyl | 5 |
| WP151 | 5 |
| Benzoate d'alcool en C12/C15 | 10 |
| Oxyde de titane | 3 |
| Triéthanolamine | Os pH : 7 |
| Glycéride | 3 |
| Conservateurs | Qs |
| Eau déminéralisée | qsp 100 |

## Revendications

**1.** Composés 5,6-diphényl-1,2,4-triaziniques de formule générale (I) :

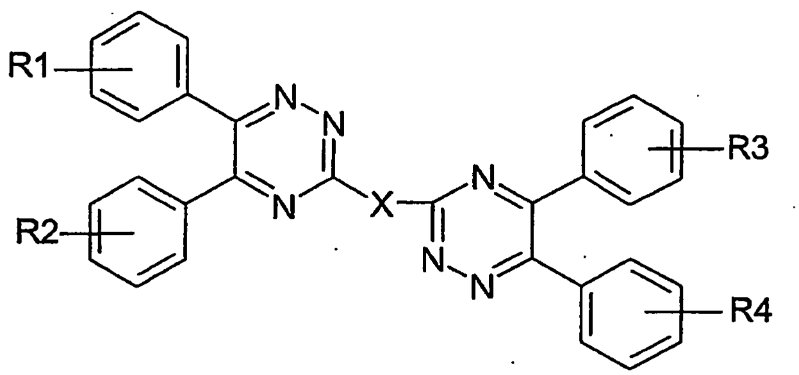

(I)

dans laquelle :

R1, R2, R3 et R4, identiques ou différents, représentent un atome d'hydrogène, de fluor, de chlore ou de brome, un groupe alkyle linéaire ou ramifié en $C_1$ à $C_{12}$, hydroxy, alcoxy linéaire ou ramifié en $C_1$ à $C_{18}$, poly(éthoxy)-alcoxy avec un fragment alkyle en $C_1$ à $C_4$ et le nombre de motif éthoxy compris entre 1 et 4, amino, ou mono- ou di-alkylamino avec un fragment alkyle en $C_1$ à $C_4$,
X représente un groupe ortho-, méta- ou para-phénylène, 4,4'-biphénylène, 2,4- ou 2,6- ou 3,4- ou 3,5-pyridinylène, 2,2'-bipyridinylène, méta- ou para-phénylènediamino, éthylènediamino, 2,2'-pipérazinylène, diacyle de formule - $(R_4CO)_2$- où $R_4$ représente un radical phényle, une chaîne alkylée de 3 à 10 atomes de carbone, phénanthrène ou anthracène,
à l'exception du 1,4-bis(5,6-diphényl-1,2,4-triazin-3-yl)benzène, de la 2,4-bis(5,6-diphényl-1,2,4-triazin-3-yl) pyridine et de la 2,6-bis(5,6-diphényl-1,2,4-triazin-3yl) pyridine.

**2.** Composés 5,6-diphényl-1,2,4-triaziniques selon la revendication 1 de formule (I), dans laquelle :

R1, R2, R3 et R4 représentent un groupe alkyle linéaire ou ramifié en $C_1$ à $C_{12}$ situé en position para, ou un groupe alcoxy linéaire ou ramifié en $C_1$ à $C_{18}$ situé en position para, et
X représente un groupe 4,4'-biphénylène.

**3.** Composés 5,6-diphényl-1,2,4-triaziniques selon les revendications 1 ou 2, choisis parmi :

WP35 : 1,4-Bis[5,6-(4,4'-dihydroxy)-diphényl-1,2,4-triazin-3-yl]benzène
WP38 : 1,4-Bis[5,6-(4,4'-di-octadécyloxy)-diphényl-1,2,4-triazin-3-yl] benzène
WP39 : 1,4-Bis[5,6-(4,4'-diméthyl)-diphényl-1,2,4-triazin-3-yl]benzène
WP40 : 1,4-Bis[5,6-(4,4'-diméthoxy)-diphényl-1,2,4-triazin-3-yl]benzène
WP41 : 1,4-Bis[5,6-(4,4'-(2-{2-[2-(2-méthoxy-éthoxy)-éthoxy]-éthoxy}-éthoxy)-)-diphényl-1,2,4-triazin-3-yl] benzène
WP52 : :1,4-Bis-[5,6-(4,4'-dibromo)-diphényl-1,2,4-triazin-3-yl]benzène
WP76 : 1,3-Bis(5,6-diphényl-1,2,4-triazin-3-yl)benzène
WP89 : 1,4-Bis[5,6-(4,4'-difluoro)-diphényl-1,2,4-triazin-3-yl]benzène
WP96 : 1,4-Bis[5,6-(4,4'-dihexyl)-diphényl-1,2,4-triazin-3-yl]benzène
WP 100 : 1,4-Bis[5,6-(4,4'-dichloro)-diphényl-1,2,4-triazin-3-yl]benzène
WP104:1,4-Bis[5,6-(4,4'-4-di-tert-butyl)-diphényl-1,2,4-triazin-3-yl]benzène
WP107 : 1,4-Bis[5,6-bis(4-dodécylphényl)-1,2,4-triazine-3-yl]benzène
WP135 : 1,4-bis-[5,6-(4,4'-diphényl-1,2,4-triazine-3-yl] diphényle
WP 144 : 1,4-bis-[5,6-bis(4,2-éthylhexyloxyphényl)-1,2,4-triazine-3-yl]benzène
WP 149 : 1,4-bis[5,6-bis(4,1-méthylheptyloxyphényl)-1,2,4-triazine-3-yl]benzène
WP 151 : bis(4-(1,1,4-triméthylpentyl)phényl)-1,2,4-triazine-3-yl]benzène.

**4.** Composition cosmétique anti-solaire active dans l'UV-A et/ou l'UV-B et/ou l'UV-C contenant une quantité efficace d'au moins un composé 5,6-diphényl-1,2,4-triazinique de formule générale (I) :

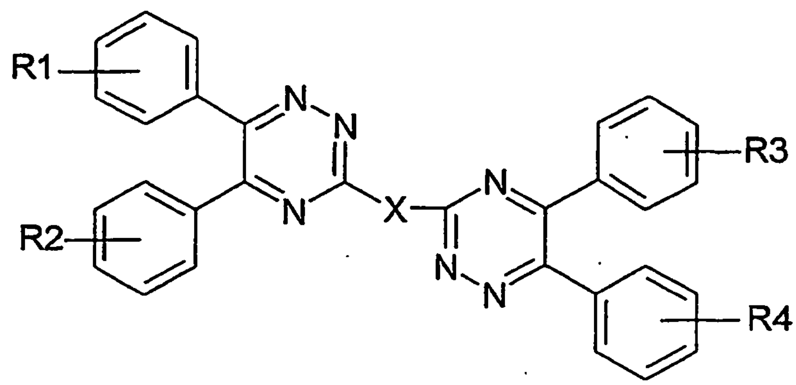

(I)

dans laquelle :

R1, R2, R3 et R4, identiques ou différents, représentent un atome d'hydrogène, de fluor, de chlore ou de brome, un groupe alkyle linéaire ou ramifié en $C_1$ à $C_{,2}$, hydroxy, alcoxy linéaire ou ramifié en $C_1$ à $C_{18}$, poly(éthoxy)-alcoxy avec un fragment alkyle en $C_1$ à $C_4$ et le nombre de motif éthoxy compris entre 1 et 4, amino, ou mono- ou di-alkylamino avec un fragment alkyle en $C_1$ à $C_4$,
X représente un groupe ortho-, méta- ou para-phénylène, 4,4'-biphénylène, 2,4- ou 2,6- ou 3,4- ou 3,5-pyridinylène, 2,2'-bipyridinylène, méta- ou para-phénylènediamino, éthylènediamino, 2,2'-pipérazinylène, diacyle de formule - $(R_4CO)_2$- où $R_4$ représente un radical phényle, une chaîne alkylée de 3 à 10 atomes de carbone, phénanthrène ou anthracène,
en association avec un excipient cosmétiquement acceptable, comprise de préférence entre 0,1 % et 20% en poids par rapport au poids total de la composition.

**5.** Composition cosmétique anti-solaire selon la revendication 4, **caractérisée en ce qu'**elle contient en outre un ou plusieurs autres filtres solaires complémentaires actifs dans l'UV-A et/ou dans l'UV-B et/ou dans l'UV-C.

**6.** Composition cosmétique anti-solaire selon la revendication 4 ou 5, **caractérisée en ce que** le composé 5,6-diphényl-1,2,4-triazinique de formule générale (I) est le 1,4-bis(5,6-diphényl-1,2,4-triazin-3-yl)benzène.

**7.** Utilisation des composés 5,6-diphényl-1,2,4-triaziniques de formule générale (I) :

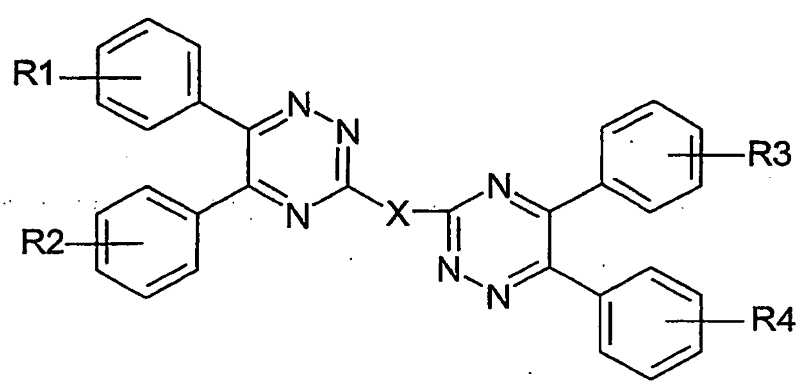

(I)

dans laquelle :

R1, R2, R3 et R4, identiques ou différents, représentent un atome d'hydrogène, de fluor, de chlore ou de brome, un groupe alkyle linéaire ou ramifié en $C_1$ à $C_{12}$, hydroxy, alcoxy linéaire ou ramifié en $C_1$ à $C_{18}$, poly(éthoxy)-alcoxy avec un fragment alkyle en $C_1$ à $C_4$ et le nombre de motif éthoxy compris entre 1 et 4, amino, ou mono- ou di-alkylamino avec un fragment alkyle en $C_1$ à $C_4$,
X représente un groupe ortho-, méta- ou para-phénylène, 4,4'-biphénylène, 2,4- ou 2,6- ou 3,4- ou 3,5-pyridinylène, 2,2'-bipyridinylène, méta- ou para-phénylènediamino, éthylènédiamino, 2,2'-pipérazinylène, diacyle de formule - $(R_4CO)_2$- où R4 représente un radical phényle, une chaîne alkylée de 3 à 10 atomes de carbone, phénanthrène ou anthracène,
comme filtres solaires actifs dans l'UV-A et/ou l'UV-B et/ou l'UV-C pour la peau humaine et/ou les cheveux.

**8.** Utilisation selon la revendication 7, **caractérisée en ce que** le composé 5,6-diphényl-1,2,4-triazinique de formule générale (I) est le 1,4-bis(5,6-diphényl-1,2,4-triazin-3-yl)benzène.

**9.** Utilisation des composés tels que définis à la revendication 7, comme agents protecteurs de la lumière actifs dans l'UV-A et/ou l'UV-B et/ou l'UV-C, utiles dans l'industrie des matériaux synthétiques.

**10.** Utilisation selon la revendication 9, comme agents protecteurs de la lumière entrant dans la composition de matières plastiques, de verre ou de matières textiles.

**11.** Utilisation selon la revendication 9 ou 10, **caractérisée en ce que** le composé 5,6-diphényl-1,2,4-triazinique de formule générale (I) est le 1,4-bis(5,6-diphényl-1,2,4-triazin-3-yl)benzène.

**Claims**

**1.** 5,6-diphenyl-1,2,4-triazinic compounds of general formula (I):

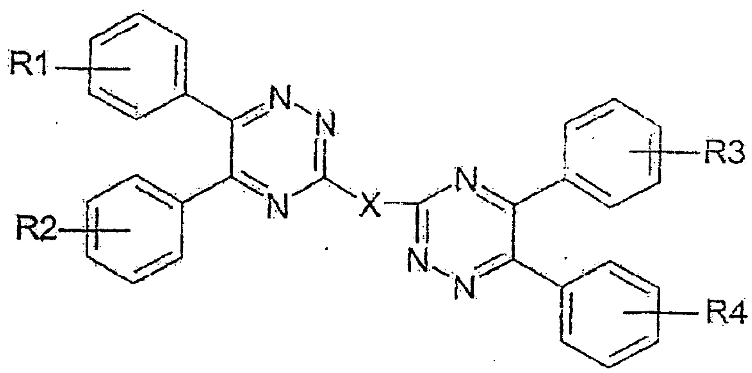

(I)

wherein:

- R1, R2, R3 and R4, identical or different, represent a hydrogen, fluorine, chlorine or bromine atom, a $C_1$ to $C_{12}$ linear or branched alkyl group, a hydroxy group, a $C_1$ to $C_{18}$ linear or branched alkoxy group, a poly (ethoxy)-alkoxy group with a $C_1$ to $C_4$ alkyl fragment and an ethoxy number ranging from 1 to 4, an amino, mono- or di-alkylamino group with a $C_1$ to $C_4$ alkyl fragment,
- X represents an ortho-, meta- or para-phenylene group, a 4,4'-biphenylene group, a 2,4- or 2,6- or 3,4- or 3,5-pyridinylene group, a 2,2'-bipyridinylene group, a meta- or para-phenylenediamino group, an ethylenediamino group, a 2,2'-piperazinylene group, a diacyl group of formula
- $(R_4CO)_2$- wherein $R_4$ represents a phenyl radical, an alkylated chain of 3 to 10 carbon atoms, phenanthrene or anthracene, with the exception of 1,4-bis(5,6-diphenyl-1,2,4-triazin-3-yl)benzene, 2,4-bis(5,6-diphenyl-1,2,4-triazin-3-yl)pyridine, and 2,6-bis(5,6-diphenyl-1,2,4-triazin-3-yl)pyridine.

**2.** The 5,6-diphenyl-1,2,4-triazinic compounds of general formula (I) according to claim 1:, wherein:

- R1, R2, R3 and R4 represent a $C_1$ to $C_{12}$ linear or branched alkyl group located in the para position, or a $C_1$ to $C_{18}$ linear or branched alkoxy group located in the para position, and,
- X represents a 4,4'-biphenylene group.

**3.** The 5,6-diphenyl-1,2,4-triazinic compounds according to claims 1 or 2, selected among:

- WP35: 1,4-Bis[5,6-(4,4'-dihydroxy)-diphenyl-1,2,4-triazin-3-yl]benzene
- WP38: 1,4-Bis[5,6-(4,4'-di-octadecyloxy)-diphenyl-1,2,4-triazin-3-yl]benzene
- WP39: 1,4-Bis[5,6-(4,4'-dimethyl)-diphenyl-1,2,4-triazin-3-yl]benzene
- WP40: 1,4-Bis[5,6-(4,4'-dimethoxy)-diphenyl-1,2,4-triazin-3-yl]benzene
- WP41: 1,4-Bis[5,6-(4,4'-[2-{2-[2-(2-methoxy-ethoxy)-ethoxy]-ethoxy}-ethoxy)-)-diphenyl-1,2,4-triazin-3-yl] benzene
- WP52: 1,4-Bis-[5,6-(4,4'-dibromo)-diphenyl-1,2,4-triazin-3-yl]benzene
- WP76: 1,3-Bis(5,6-diphenyl-1,2,4-triazin-3-yl)benzene
- WP89: 1,4-Bis[5,6-(4,4'-difluoro)-diphenyl-1,2,4-triazin-3-yl]benzene
- WP96: 1,4-Bis[5,6-(4,4'-dihexyl)-diphenyl-1,2,4-triazin-3-yl]benzene
- WP100: 1,4-Bis[5,6-(4,4'-dichloro)-diphenyl-1,2,4-triazin-3-yl]benzene
- WP104: 1,4-Bis[5,6-(4,4'-4-di-tert-butyl)-diphenyl-1,2,4-triazin-3-yl]benzene
- WP107: 1,4-Bis[5,6-bis(4-dodecylphenyl)-1,2,4-triazine-3-yl]benzene
- WP135: 1,4-bis-[5,6-(4,4'-diphenyl-1,2,4-triazine-3-yl]diphenyl
- WP144: 1,4-bis-[5,6-bis(4,2-ethylhexyloxyphenyl)-1,2,4-triazine-3-yl]benzene
- WP149: 1,4-bis[5,6-bis(4,1-methylheptyloxyphenyl)-1,2,4-triazine-3-yl]-benzene
- WP151: bis(4-(1,1,4-trimethylpentyl)phenyl)-1,2,4-triazine-3-yl]benzene.

**4.** A cosmetic sunscreen composition active in UV-A and/or UV-B and/or UV-C containing an effective quantity of at least one 5,6-diphenyl-1,2,4-triazinic compound of general formula (I):

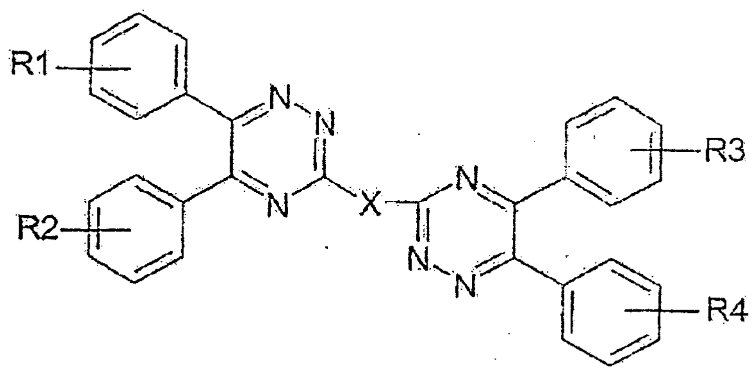

(I)

wherein:

- R1, R2, R3 and R4, identical or different, represent a hydrogen, fluorine, chlorine or bromine atom, a $C_1$ to $C_{12}$ linear or branched alkyl group, a hydroxy group, a $C_1$ to $C_{18}$ linear or branched alkoxy group, a poly (ethoxy)-alkoxy group with a $C_1$ to $C_4$ alkyl fragment and an ethoxy number ranging from 1 to 4, an amino, mono- or di-alkylamino group with a $C_1$ to $C_4$ alkyl fragment,
- X represents an ortho-, meta- or para-phenylene group, a 4,4'-biphenylene group, a 2,4- or 2,6- or 3,4- or 3,5-pyridinylene group, a 2,2'-bipyridinylene group, a meta- or para-phenylenediamino group, an ethylenediamino group, a 2,2'-piperazinylene group, a diacyl group of formula
- $(R_4CO)_2$- wherein $R_4$ represents a phenyl radical, an alkylated chain of 3 to 10 carbon atoms, phenanthrene or anthracene, in combination with a cosmetically acceptable excipient, preferably comprised between 0.1% and 20% by weight with respect to the total weight of the composition.

**5.** The cosmetic sunscreen composition according to claim 4, **characterized in that** it contains in addition one or more complementary sun filters active in UV-A and/or UV-B and/or UV-C.

6. The cosmetic sunscreen composition according to claims 4 or 5, **characterised in that** the 5,6-diphenyl-1,2,4-triazinic compound of general formula (I) is 1,4-bis(5,6-diphenyl-1,2,4-triazin-3-yl)benzene.

7. The use of 5,6-diphenyl-1,2,4-triazinic compounds of general formula (I):

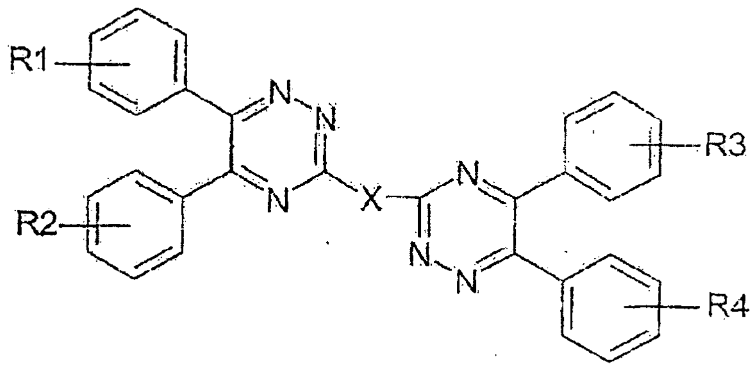

(I)

wherein:

- R1, R2, R3 and R4, identical or different, represent a hydrogen, fluorine, chlorine or bromine atom, a $C_1$ to $C_{12}$ linear or branched alkyl group, a hydroxy group, a $C_1$ to $C_{18}$ linear or branched alkoxy group, a poly (ethoxy)-alkoxy group with a $C_1$ to $C_4$ alkyl fragment and an ethoxy number ranging from 1 to 4, an amino, mono- or di-alkylamino group with a $C_1$ to $C_4$ alkyl fragment,
- X represents an ortho-, meta- or para-phenylene group, a 4,4'-biphenylene group, a 2,4- or 2,6- or 3,4- or 3,5-pyridinylene group, a 2,2'-bipyridinylene group, a meta- or para-phenylenediamino group, an ethylenediamino group, a 2,2'-piperazinylene group, a diacyl group of formula
- $(R_4CO)_2$- wherein $R_4$ represents a phenyl radical, an alkylated chain of 3 to 10 carbon atoms, phenanthrene or anthracene

as sunscreens active in UV-A and/or UV-B and/or UV-C for human skin and/or hair.

8. The use according to claim 7, **characterised in that** the 5,6-diphenyl-1,2,4-triazinic compound of general formula (I) is 1,4-bis(5,6-diphenyl-1,2,4-triazin-3-yl)benzene.

9. The use of compounds as defined in claim 7, as light-protective agents active in UV-A and/or UV-B and/or UV-C, useful in the synthetic material industry.

10. The use according to claim 9, as light-protective agents incorporated into the composition of plastics, glass or textiles.

11. The use according to claims 9 or 10, **characterised in that** the 5,6-diphenyl-1,2,4-triazinic compound of general formula (I) is 1,4-bis(5,6-diphenyl-1,2,4-triazin-3-yl)benzene.

**Patentansprüche**

1. 5,6-Diphenyl-1,2,4-triazin-Verbindungen der allgemeinen Formel (I):

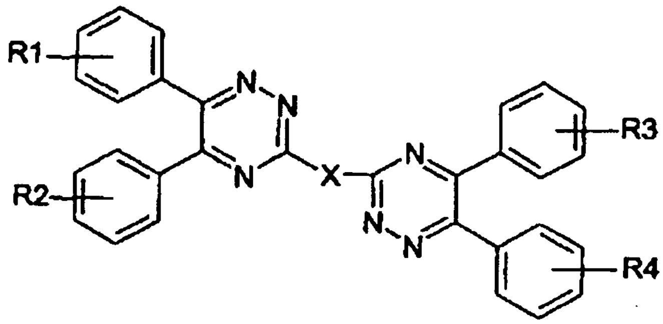

(I)

,

in welcher:

$R_1$, $R_2$, $R_3$ und $R_4$, die gleich oder verschieden sind, für ein Wasserstoff-, Fluor-, Chlor- oder Bromatom, eine lineare oder verzweigte $C_1$- bis $C_{12}$-Alkylgruppe, eine Hydroxygruppe, eine lineare oder verzweigte $C_1$- bis $C_{18}$-Alkoxygruppe, eine Poly(ethoxy)alkoxygruppe mit einem $C_1$- bis $C_4$-Alkylfragment und mit einer Anzahl von Ethoxy-Struktureinheiten zwischen 1 und 4 einschließlich, eine Aminogruppe oder eine Mono- oder Dialkylaminogruppe mit einem $C_1$- bis $C_4$-Alkylfragment stehen,

X darstellt: eine ortho-, meta- oder para-Phenylengruppe, 4,4'-Biphenylengruppe, 2,4- oder 2,6- oder 3,4- oder 3,5-Pyridinylengruppe, 2,2'-Bipyridinylengruppe, meta- oder para-Phenylendiaminogruppe, Ethylendiaminogruppe, 2,2'-Piperazinylengruppe, eine Diacylgruppe der Formel $-(R_4CO)_2-$, worin $R_4$ darstellt: einen Phenylrest, eine Alkylkette mit 3 bis 10 Kohlenstoffatomen, Phenanthren oder Anthracen,

mit der Ausnahme von 1,4-Bis(5,6-diphenyl-1,2,4-triazin-3-yl)-benzol, 2,4-Bis(5,6-diphenyl-1,2,4-triazin-3-yl)pyridin und 2,6-Bis(5,6-diphenyl-1,2,4-triazin-3-yl)pyridin.

**2.** 5,6-Diphenyl-1,2,4-triazin-Verbindungen nach Anspruch 1 der Formel (I), in welcher:

$R_1$, $R_2$, $R_3$ und $R_4$ darstellt: eine lineare oder verzweigte $C_1$-bis $C_{12}$-Alkylgruppe, die sich in para-Position befindet, oder eine lineare oder verzweigte $C_1$-bis $C_{18}$-Alkoxygruppe, die sich in para-Position befindet, und X eine 4,4'-Biphenylengruppe darstellt.

**3.** 5,6-Diphenyl-1,2,4-triazin-Verbindungen nach den Ansprüchen 1 oder 2, die ausgewählt sindaus:

WP35: 1,4-Bis[5,6-(4,4'-dihydroxy)-diphenyl-1,2,4-triazin-3-yl]benzol,
WP38: 1,4-Bis[5,6-(4,4'dioctadecyloxy)diphenyl-1,2,4-triazin-3-yl]benzol,
WP39: 1,4-Bis[5,6-(4,4'-dimethyl)diphenyl-1,2,4-triazin-3-yl]benzol,
WP40: 1,4-Bis[5,6-(4,4'-dimethoxy)diphenyl-1,2,4-triazin-3-yl]benzol,
WP41: 1,4-Bis[5,6-(4,4'-(2-{2-[2-(2-methoxyethoxy)ethoxyl]ethoxy}ethoxy)-)-diphenyl-1,2,4-triazin-3-yl]benzol,
WP52: 1,4-Bis[5,6-(4,4'-dibrom)diphenyl-1,2,4-triazin-3-yl]benzol,
WP76: 1,3-Bis(5,6-diphenyl-1,2,4-triazin-3-yl)benzol,
WP89: 1,4-Bis[5,6-(4,4'-difluor)diphenyl-1,2,4-triazin-3-yl]benzol,
WP96: 1,4-Bis[5,6-(4,4'-dihexyl)diphenyl-1,2,4-triazin-3-yl]benzol,
WP100: 1,4-Bis[5,6-(4,4'-dichlor)diphenyl-1,2,4-triazin-3-yl]benzol,
WP104: 1,4-Bis[5,6-(4,4'-4-di-*tert*-butyl)diphenyl-1,2,4-triazin-3-yl]benzol,
WP107: 1,4-Bis[5,6-bis(4-dodecylphenyl)-1,2,4-triazin-3-yl]benzol,
WP135: 1,4-Bis[5,6-(4,4'-diphenyl-1,2,4-triazin-3-yl]diphenyl,
WP144: 1,4-Bis[5,6-bis(4,2-ethylhexyloxyphenyl)-1,2,4-triazin-3-yl]benzol,
WP149: 1,4-Bis[5,6-bis(4,1-methylheptyloxyphenyl)-1,2,4-triazin-3-yl]benzol,
WP151: Bis(4-(1,1,4-trimethylpentyl)phenyl)-1,2,4-triazin-3-yl]benzol.

**4.** Kosmetische Sonnenschutzzusammensetzung, die im UV-A- und/oder UV-B- und/oder UV-C-Bereich aktiv ist, enthaltend eine wirksame Menge mindestens einer 5,6-Diphenyl-1,2,4-triazin-Verbindung der allgemeinen Formel (I):

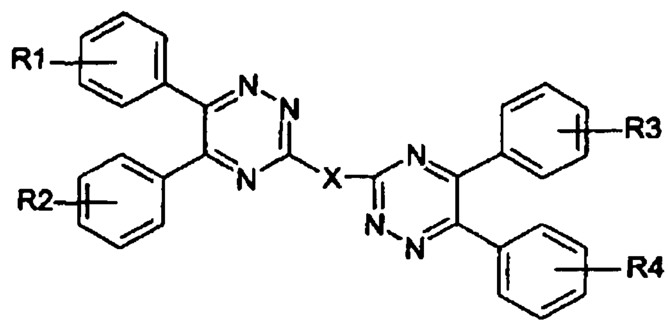

(I) ,

in welcher:

$R_1$, $R_2$, $R_3$ und $R_4$, die gleich oder verschieden sind, für ein Wasserstoff-, Fluor-, Chlor- oder Bromatom, eine lineare oder verzweigte $C_1$- bis $C_{12}$-Alkylgruppe, eine Hydroxygruppe, eine lineare oder verzweigte $C_1$- bis $C_{18}$-Alkoxygruppe, eine Poly(ethoxy)alkoxygruppe mit einem $C_1$- bis $C_4$-Alkylfragment und mit einer Anzahl von Ethoxy-Struktureinheiten zwischen 1 und 4 einschließlich, eine Aminogruppe oder eine Mono- oder Dialkylaminogruppe mit einem $C_1$- bis $C_4$-Alkylfragment stehen,
X darstellt: eine ortho-, meta- oder para-Phenylengruppe, 4,4'-Biphenylengruppe, 2,4- oder 2,6- oder 3,4- oder 3,5-Pyridinylengruppe, 2,2'-Bipyridinylengruppe, meta- oder para-Phenylendiaminogruppe, Ethylendiaminogruppe, 2,2'-Piperazinylengruppe, eine Diacylgruppe der Formel -$(R_4CO)_2$-, worin $R_4$ darstellt: einen Phenylrest, eine Alkylkette mit 3 bis 10 Kohlenstoffatomen, Phenanthren oder Anthracen,
in Kombination mit einem kosmetisch annehmbaren Träger, welcher vorzugsweise in einer Konzentration zwischen 0,1 Gew.-% und 20 Gew.-% einschließlich, bezogen auf das Gesamtgewicht der Zusammensetzung, enthalten ist.

5. Kosmetische Sonnenschutzzusammensetzung nach Anspruch 4, **dadurch gekennzeichnet, dass** sie außerdem einen oder mehrere andere ergänzende Sonnenfilter enthält, die im UV-A- und/oder UV-B- und/oder UV-C-Bereich aktiv sind.

6. Kosmetische Sonnenschutzzusammensetzung nach Ansprüchen 4 oder 5, **dadurch gekennzeichnet, dass** der 5,6-diphenyl-1,2,4-triazin- Verbindungen der algemeinen formel (I) 1,4-Bis(5,6-diphenyl-1,2,4-triazin-3-yl)benzol ist.

7. Verwendung von 5,6-Diphenyl-1,2,4-triazin-Verbindungen der allgemeinen Formel (I):

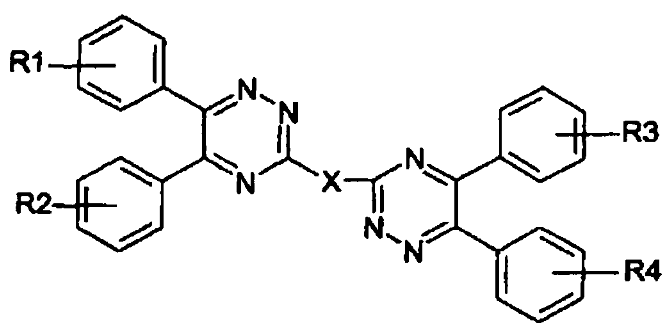

(I) ,

in welcher:

$R_1$, $R_2$, $R_3$ und $R_4$, die gleich oder verschieden sind, für ein Wasserstoff-, Fluor-, Chlor- oder Bromatom, eine lineare oder verzweigte $C_1$- bis $C_{12}$-Alkylgruppe, eine Hydroxygruppe, eine lineare oder verzweigte $C_1$- bis

$C_{18}$-Alkoxygruppe, eine Poly(ethoxy)alkoxygruppe mit einem $C_1$- bis $C_4$-Alkylfragment und mit einer Anzahl von Ethoxy-Struktureinheiten zwischen 1 und 4 einschließlich, eine Aminogruppe oder eine Mono- oder Dialkylaminogruppe mit einem $C_1$- bis $C_4$-Alkylfragment stehen,
X darstellt: eine ortho-, meta- oder para-Phenylengruppe, 4,4'-Biphenylengruppe, 2,4- oder 2,6- oder 3,4- oder 3,5-Pyridinylengruppe, 2,2'-Bipyridinylengruppe, meta- oder para-Phenylendiaminogruppe, Ethylendiaminogruppe, 2,2'-Piperazinylengruppe, eine Diacylgruppe der Formel $-(R_4CO)_2-$, worin $R_4$ darstellt: einen Phenylrest, eine Alkylkette mit 3 bis 10 Kohlenstoffatomen, Phenanthren oder Anthracen,
als Sonnenfilter, die im UV-A- und/oder UV-B- und/oder UV-C-Bereich aktiv sind, für die menschliche Haut und/oder die Haare.

**8.** Verwendung nach Anspruch 7, **dadurch gekennzeichnet, dass** die 5,6-Diphenyl-1,2,4-triazin-Verbindung der allgemeinen Formel (I) 1,4-Bis(5,6-diphenyl-1,2,4-triazin-3-yl)benzol ist.

**9.** Verwendung der Verbindungen wie Anspruch 7 definiert, als Lichtschutzmittel, die im UV-A- und/oder UV-B- und/oder UV-C-Bereich aktiv sind, welche im Industriezweig der synthetischen Materialien nützlich sind.

**10.** Verwendung nach Anspruch 9 als Lichtschutzmittel, die in die Zusammensetzung von Kunststoffmaterialien, von Glas oder von Textilmaterialien Eingang finden.

**11.** Verwendung nach Anspruch 9 oder 10, **dadurch gekennzeichnet, dass** die 5,6-Diphenyl-1,2,4-triazin-Verbindung der allgemeinen Formel (I) 1,4-Bis(5,6-diphenyl-1,2,4-triazin-3-yl)benzol ist.

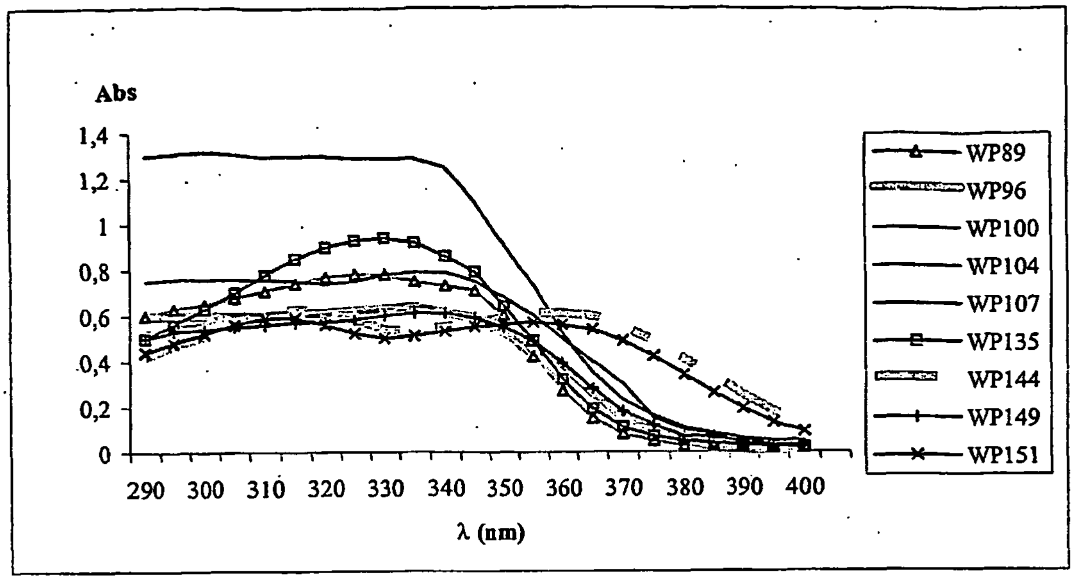


FIGURE 1

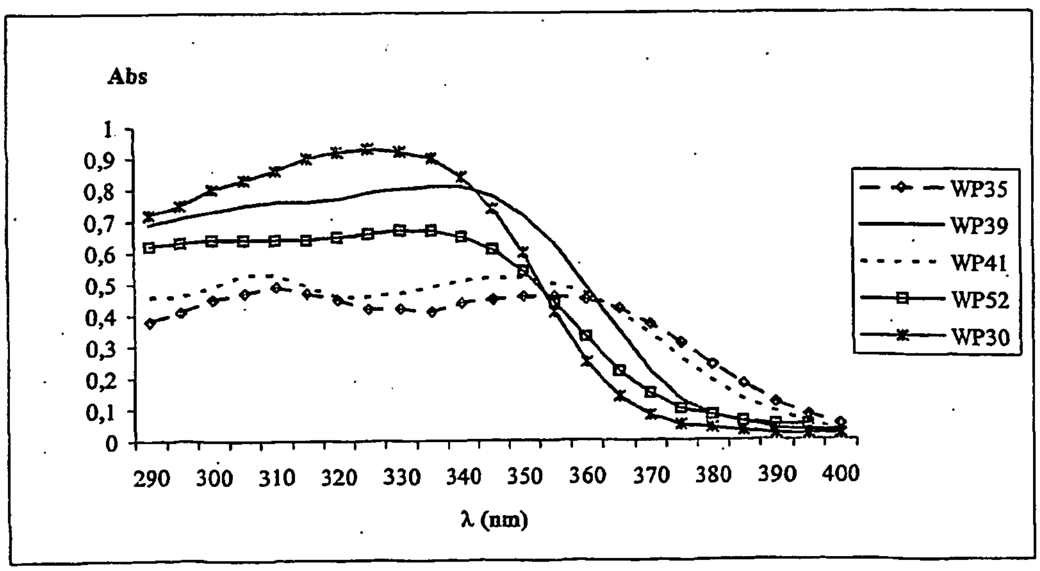


FIGURE 2

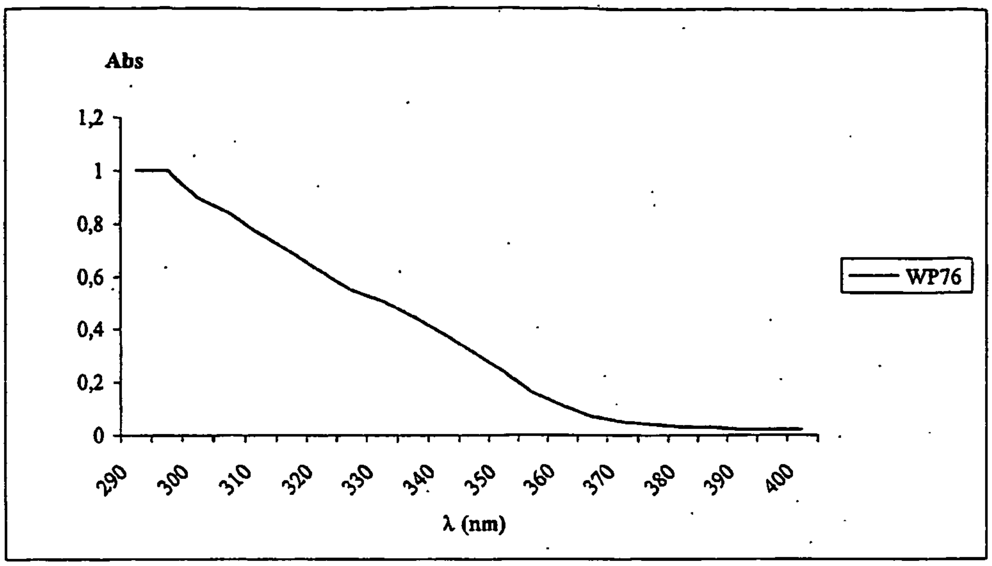
Abs
1,2
1
0,8
0,6
0,4
0,2
0
290
300
310
320
330
340
350
360
370
380
390
400
λ (nm)
WP76

FIGURE 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2803194
- JP 04279546 B
- JP 43015992 B
- US 5202471 A


### Littérature non-brevet citée dans la description


- **G. Pitet ; H. Cousse ; G. Mouzin.** *Boll, Chim, Farm.,* 1980, vol. 119, 469
- **F. Lu ; Y. Wang ; L. Xing.** *Gaofenzi Tongxun,* 1981, vol. 5, 319
- **X. Yi ; G. Wu ; F. Lu ; A. Tang.** *J. Appl. Point. Sci.,* 2001, vol. 907, 82
- **H. Simbürger ; W. Kern ; K. Hummel ; C. Hagg.** *Polym.,* 2000, vol. 41, 7883
- **L. Brunsvelt ; H. Zhang ; M. Glasbeek ; J. A. J. M. Vekemans ; E. W. Meijer.** *J. Am. Chem. Soc.,* 2000, vol. 122, 6175
- **M. Wehmeier ; M. Wagner ; K. Müllen.** *Chem. Eur. J.,* 2001, vol. 7, 2197
- **M. S. Alnajjar ; H. G. Kuivila.** *J. Am. Chem. Soc.,* 1985, vol. 107, 422